# FASCICULE DE BREVET EUROPEEN

(11) **EP 1 442 124 B1**
(45) Date de publication et mention de la délivrance du brevet: **20.12.2006**
(21) Numéro de dépôt: 02783174.2
(22) Date de dépôt: 18.09.2002
(51) Int. Cl.: C12N 15/49, C12N 15/62, C12N 15/85, C12N 1/21, C07K 14/16, A61K 39/21, A61P 31/18

(54) **ANTIGENE POLYPEPTIDIQUE FORMANT UNE STRUCTURE MIMANT L'ETAT INTERMEDIAIRE DE gp41**
EINE DEN GP41-ZWISCHENZUSTAND IMITIERENDE STRUKTUR BILDENDES POLYPEPTID-ANTIGEN
POLYPEPTIDE ANTIGEN FORMING A MIMETIC STRUCTURE OF THE GP41 INTERMEDIATE STATE

(30) Priorité: 05.10.2001 FR 0112806; 07.11.2001 US 347909 P
(43) Date de publication de la demande: 04.08.2004
(73) Titulaire: Sanofi Pasteur, 69367 Lyon Cedex 07 (FR)
(72) Inventeur: CHEVALIER, Michel, F-38270 Beaurepaire (FR); EL HABIB, Raphaelle, F-69630 Chaponost (FR); KRELL, Tino, F-69130 Ecully (FR); SODOYER, Régis, F-69110 Sainte Foy les Lyon (FR)
(74) Mandataire: Schaeffer, Nathalie Christiane
(86) Numéro de dépôt international: PCT/FR2002/003188
(87) Numéro de publication internationale: WO 2003/042388

(56) Documents cités:
- WO-A-00/08167
- WO-A-00/40616
- WO-A-01/44286
- WO-A-01/70262
- SHU W. ET AL.: "Helical interactions in the HIV-1 gp41 core reveals structural basis for the inhibitory activity of gp41 peptides" BIOCHEMISTRY, vol. 39, 2000, pages 1634-1642, XP002201625
- LOUIS J.M. ET AL.: "Design and properties of Nccg-gp41, a chimeric gp41 molecule with nanomolar HIV fusion inhibitory activity." J. BIOL. CHEM., vol. 276, no. 31, 3 août 2001 (2001-08-03), pages 29485-29489, XP002201626

### SEQUENCE LISTING

<110> AVENTIS PASTEUR
<120> Antigène polypeptidique formant une structure mimant l'état intermédiaire de gp41
<130> PM0106 WO
<160> 35
<170> PatentIn version 3.1
<210> 1
   <211> 29
   <212> DNA
   <213> Artificial
<220>
   <223> amorce
<400> 1
   catgccatgg ccagacaatt attgtctgg 29
<210> 2
   <211> 42
   <212> DNA
   <213> Artificial
<220>
   <223> amorce
<400> 2
   ctccatccag gtcatgttat tatcctttag gtatctttcc ac 42
<210> 3
   <211> 42
   <212> DNA
   <213> Artificial
<220>
   <223> amorce
<400> 3
   gtggaaagat acctaaagga taataacatg acctggatgg ag 42
<210> 4
   <211> 66
   <212> DNA
   <213> Artificial
<220>
   <223> amorce
<400> 4
   ccgctcgagc taatggtgat ggtgatggtg tgaccctccc cctcctttat ctaattccaa 60
   taattc 66
<210> 5
   <211> 29
   <212> DNA
   <213> Artificial
<220>
<400> 5
   catgccatgg ccagacaatt attgtctgg 29
<210> 6
   <211> 47
   <212> DNA
   <213> Artificial
<220>
   <223> amorce
<400> 6
   gttaatttct ctgtcccact ccatccactg ttgatccttt aggtatc 47
<210> 7
   <211> 47
   <212> DNA
   <213> Artificial
<220>
   <223> amorce
<400> 7
   gatacctaaa ggatcaacag tggatggagt gggacagaga aattaac 47
<210> 8
   <211> 66
   <212> DNA
   <213> Artificial
<220>
   <223> amorce
<400> 8
   ccgctcgagc taatggtgat ggtgatggtg tgaccctccc cctcctttat ctaattccaa 60
   taattc 66
<210> 9
   <211> 29
   <212> DNA
   <213> Artificial
<220>
   <223> amorce
<400> 9
   catgccatgg ccagacaatt attgtctgg 29
<210> 10
   <211> 36
   <212> DNA
   <213> Artificial
<220>
   <223> amorce
<400> 10
   catccaggtc atgttattat cctttaggta tctttc 36
<210> 11
   <211> 36
   <212> DNA
   <213> Artificial
<220>
   <223> amorce
<400> 11
   gaaagatacc taaaggataa taacatgacc tggatg 36
<210> 12
   <211> 65
   <212> DNA
   <213> Artificial
<220>
   <223> amorce
<400> 12
   ccgctcgagc taatggtgat ggtgatggtg tgaccctccc cctcctttat ctaattccaa 60
   taatt 65
<210> 13
   <211> 36
   <212> DNA
   <213> Artificial
<220>
   <223> amorce
<400> 13
   ttattggaat tagataaagc cagacaatta ttgtct 36
<210> 14
   <211> 63
   <212> DNA
   <213> Artificial
<220>
   <223> amorce
<400> 14
   ccgctcgagc taatggtgat ggtgatggtg tgaccctccc cctcccttta ggtatctttc 60
   cac 63
<210> 15
   <211> 31
   <212> DNA
   <213> Artificial
<220>
   <223> amorce
<400> 15
   catgccatgg gatggatgga gtgggacaga g 31
<210> 16
   <211> 36
   <212> DNA
   <213> Artificial
<220>
   <223> amorce
<400> 16
   agacaataat tgtctggctt tatctaattc caataa 36
<210> 17
   <211> 39
   <212> DNA
   <213> Artificial
<220>
   <223> amorce
<400> 17
   ggaattagat aaatgggcag ccagacaatt attgtctgg 39
<210> 18
   <211> 63
   <212> DNA
   <213> Artificial
<220>
   <223> amorce
<400> 18
   ccgctcgagc taatggtgat ggtgatggtg tgaccctccc cctcccttta ggtatctttc 60
   cac 63
<210> 19
   <211> 31
   <212> DNA
   <213> Artificial
<220>
   <223> amorce
<400> 19
   catgccatgg gatggatgga gtgggacaga g 31
<210> 20
   <211> 39
   <212> DNA
   <213> Artificial
<220>
   <223> amorce
<400> 20
   ccagacaata attgtctggc tgcccattta tctaattcc 39
<210> 21
   <211> 40
   <212> DNA
   <213> Artificial
<220>
   <223> amorce
<400> 21
   gggcaagttt gtggaattgg gccagacaat tattgtctgg 40
<210> 22
   <211> 63
   <212> DNA
   <213> Artificial
<220>
   <223> amorce
<400> 22
   ccgctcgagc taatggtgat ggtgatggtg tgaccctccc cctcccttta ggtatctttc 60
   cac 63
<210> 23
   <211> 31
   <212> DNA
   <213> Artificial
<220>
   <223> amorce
<400> 23
   catgccatgg gatggatgga gtgggacaga g 31
<210> 24
   <211> 40
   <212> DNA
   <213> Artificial
<220>
   <223> amorce
<400> 24
   ccagacaata attgtctggc ccaattccac aaacttgccc 40
<210> 25
   <211> 200
   <212> PRT
   <213> Artificial
<220>
   <223> polypeptide
<400> 25
   Ala Val Gly Ile Gly Ala Leu Phe Leu Gly Phe Leu Gly Ala Ala Gly
   1               5                   10                  15
   Ser Thr Met Gly Ala Ala Ser Met Thr Leu Thr Val Gln Ala Arg Gln
               20                  25                  30
   Leu Leu Ser Gly Ile Val Gln Gln Gln Asn Asn Leu Leu Arg Ala Ile
           35                  40                  45
   Glu Ala Gln Gln His Leu Leu Gln Leu Thr Val Trp Gly Ile Lys Gln
       50                  55                  60
   Leu Gln Ala Arg Ile Leu Ala Val Glu Arg Tyr Leu Lys Asp Gln Gln
   65                  70                  75                  80
   Leu Leu Gly Ile Trp Gly Cys Ser Gly Lys Leu Ile Cys Thr Thr Ala
                   85                  90                  95
   Val Pro Trp Asn Ala Ser Trp Ser Asn Lys Ser Leu Glu Gln Ile Trp
               100                 105                 110
   Asn Asn Met Thr Trp Met Glu Trp Asp Arg Glu Ile Asn Asn Tyr Thr
           115                 120                 125
   Ser Leu Ile His Ser Leu Ile Glu Glu Ser Gln Asn Gln Gln Glu Lys
       130                 135                 140
   Asn Glu Gln Glu Leu Leu Glu Leu Asp Lys Trp Ala Ser Leu Trp Asn
   145                 150                 155                 160
   Trp Phe Asn Ile Thr Asn Trp Leu Trp Tyr Ile Lys Asn Arg Val Arg
                   165                 170                 175
   Gln Gly Tyr Ser Pro Leu Ser Phe Gln Thr His Leu Pro Thr Pro Arg
               180                 185                 190
   Gly Pro Asp Arg Pro Glu Gly Ile
           195                 200
<210> 26
   <211> 48
   <212> PRT
   <213> Artificial
<220>
   <223> polypeptide
<400> 26
   Ala Arg Gln Leu Leu Ser Gly Ile Val Gln Gln Gln Asn Asn Leu Leu
   1 5 10 15
   Arg Ala Ile Glu Ala Gln Gln His Leu Leu Gln Leu Thr Val Trp Gly
   20 25 30
   Ile Lys Gln Leu Gln Ala Arg Ile Leu Ala Val Glu Arg Tyr Leu Lys
   35 40 45
<210> 27
   <211> 38
   <212> PRT
   <213> Artificial
<220>
   <223> polypeptide
<400> 27
   Trp Met Glu Trp Asp Arg Glu Ile Asn Asn Tyr Thr Ser Leu Ile His
   1 5 10 15
   Ser Leu Ile Glu Glu Ser Gln Asn Gln Gln Glu Lys Asn Glu Gln Glu
   20 25 30
   Leu Leu Glu Leu Asp Lys
   35
<210> 28
   <211> 105
   <212> PRT
   <213> Artificial
<220>
   <223> polypeptide
<400> 28
   Met Ala Arg Gln Leu Leu Ser Gly Ile Val Gln Gln Gln Asn Asn Leu
   1 5 10 15
   Leu Arg Ala Ile Glu Ala Gln Gln His Leu Leu Gln Leu Thr Val Trp
   20 25 30
   Gly Ile Lys Gln Leu Gln Ala Arg Ile Leu Ala Val Glu Arg Tyr Leu
   35 40 45
   Lys Asp Asn Asn Met Thr Trp Met Glu Trp Asp Arg Glu Ile Asn Asn
   50 55 60
   Tyr Thr Ser Leu Ile His Ser Leu Ile Glu Glu Ser Gln Asn Gln Gln
   65 70 75 80
   Glu Lys Asn Glu Gln Glu Leu Leu Glu Leu Asp Lys Gly Gly Gly Gly
   85 90 95
   Ser His His His His His His Leu Glu
   100 105
<210> 29
   <211> 103
   <212> PRT
   <213> Artificial
<220>
   <223> polypeptide
<400> 29
   Met Ala Arg Gln Leu Leu Ser Gly Ile Val Gln Gln Gln Asn Asn Leu
   1 5 10 15
   Leu Arg Ala Ile Glu Ala Gln Gln His Leu Leu Gln Leu Thr Val Trp
   20 25 30
   Gly Ile Lys Gln Leu Gln Ala Arg Ile Leu Ala Val Glu Arg Tyr Leu
   35 40 45
   Lys Asp Gln Gln Trp Met Glu Trp Asp Arg Glu Ile Asn Asn Tyr Thr
   50 55 60
   Ser Leu Ile His Ser Leu Ile Glu Glu Ser Gln Asn Gln Gln Glu Lys
   65 70 75 80
   Asn Glu Gln Glu Leu Leu Glu Leu Asp Lys Gly Gly Gly Gly Ser His
   85 90 95
   His His His His His Leu Glu
   100
<210> 30
   <211> 105
   <212> PRT
   <213> Artificial
<220>
   <223> polypeptide
<400> 30
   Met Ala Arg Gln Leu Leu Ser Gly Ile Val Gln Gln Gln Asn Asn Leu
   1               5                   10                  15
   Leu Arg Ala Ile Glu Ala Gln Gln His Leu Leu Gln Leu Thr Val Trp
               20                  25                  30
   Gly Ile Lys Gln Leu Gln Ala Arg Ile Leu Ala Val Glu Arg Tyr Leu
           35                  40                  45
   Lys Asp Asn Asn Met Thr Trp Met Glu Trp Asp Arg Glu Ile Asn Asn
       50                  55                  60
   Tyr Thr Ser Leu Ile His Ser Leu Ile Glu Glu Ser Gln Asn Gln Gln
   65                  70                  75                  80
   Glu Lys Asn Glu Gln Glu Leu Leu Glu Leu Asp Lys Gly Gly Gly Gly
                   85                  90                  95
   Ser His His His His His His Leu Glu
               100                 105
<210> 31
   <211> 101
   <212> PRT
   <213> Artificial
<220>
   <223> polypeptide
<400> 31
   Met Gly Trp Met Glu Trp Asp Arg Glu Ile Asn Asn Tyr Thr Ser Leu
   1               5                   10                  15
   Ile His Ser Leu Ile Glu Glu Ser Gln Asn Gln Gln Glu Lys Asn Glu
               20                  25                  30
   Gln Glu Leu Leu Glu Leu Asp Lys Ala Arg Gln Leu Leu Ser Gly Ile
           35                  40                  45
   Val Gln Gln Gln Asn Asn Leu Leu Arg Ala Ile Glu Ala Gln Gln His
   50 55 60
   Leu Leu Gln Leu Thr Val Trp Gly Ile Lys Gln Leu Gln Ala Arg Ile
   65 70 75 80
   Leu Ala Val Glu Arg Tyr Leu Lys Gly Gly Gly Gly Ser His His His
   85 90 95
   His His His Leu Glu
   100
<210> 32
   <211> 103
   <212> PRT
   <213> Artificial
<220>
   <223> polypeptide
<400> 32
   Met Gly Trp Met Glu Trp Asp Arg Glu Ile Asn Asn Tyr Thr Ser Leu
   1 5 10 15
   Ile His Ser Leu Ile Glu Glu Ser Gln Asn Gln Gln Glu Lys Asn Glu
   20 25 30
   Gln Glu Leu Leu Glu Leu Asp Lys Trp Ala Ala Arg Gln Leu Leu Ser
   35 40 45
   Gly Ile Val Gln Gln Gln Asn Asn Leu Leu Arg Ala Ile Glu Ala Gln
   50 55 60
   Gln His Leu Leu Gln Leu Thr Val Trp Gly Ile Lys Gln Leu Gln Ala
   65 70 75 80
   Arg Ile Leu Ala Val Glu Arg Tyr Leu Lys Gly Gly Gly Gly Ser His
   85 90 95
   His His His His His Leu Glu
   100
<210> 33
   <211> 108
   <212> PRT
   <213> Artificial
<220>
   <223> polypeptide
<400> 33
   Met Gly Trp Met Glu Trp Asp Arg Glu Ile Asn Asn Tyr Thr Ser Leu
   1 5 10 15
   Ile His Ser Leu Ile Glu Glu Ser Gln Asn Gln Gln Glu Lys Asn Glu
   20 25 30
   Gln Glu Leu Leu Glu Leu Asp Lys Trp Ala Ser Leu Trp Asn Trp Ala
   35 40 45
   Arg Gln Leu Leu Ser Gly Ile Val Gln Gln Gln Asn Asn Leu Leu Arg
   50 55 60
   Ala Ile Glu Ala Gln Gln His Leu Leu Gln Leu Thr Val Trp Gly Ile
   65 70 75 80
   Lys Gln Leu Gln Ala Arg Ile Leu Ala Val Glu Arg Tyr Leu Lys Gly
   85 90 95
   Gly Gly Gly Ser His His His His His His Leu Glu
   100 105
<210> 34
   <211> 552
   <212> DNA
   <213> Artificial
<220>
   <223> région core de gp41
<400> 34
   atgacgctga cggtacaggc cagacaatta ttgtctggta tagtgcagca gcagaacaat 60
   ttgctgaggg ctattgaggc gcaacagcat ctgttgcaac tcacagtctg gggcatcaag 120
   cagctccagg caagaatcct ggctgtggaa agatacctaa aggatcaaca gctcctgggg 180
   atttggggtt gctctggaaa actcatttgc accactgctg tgccttggaa tgctagttgg 240
   agtaataaat ctctggaaca gatttggaat aacatgacct ggatggagtg ggacagagaa 300
   attaacaatt acacaagctt aatacattcc ttaattgaag aatcgcaaaa ccagcaagaa 360
   aagaatgaac aagaattatt ggaattagat aaatgggcaa gtttgtggaa ttggtttaac 420
   ataacaaatt ggctgtggta tataaaaaat agagttaggc agggatattc accattatcg 480
   tttcagaccc acctcccaac cccgagggga cccgacaggc ccgaaggagg tagagagcga 540
   gtccgagacc ga 552
<210> 35
   <211> 5312
   <212> DNA
   <213> Artificial
<220>
   <223> plasmide pET-cer
<400> 35
   tggcgaatgc cttaattaag gcggggcaca actcaatttg cgggtactga ttaccgcagc 60
   aaagacctta ccccgaaaaa atccaggctg ctggctgaca cgatttctgc ggtttatctc 120
   gatggctacg agggcagaca gtaagtggat ttaccataat cccttaattg tacgcaccgc 180
   taaaacgcgt tcagcgcgat cacggcagca gacaggtaaa aatggcaaca aaccacccga    240
   aaaactgccg cgatcgcgcc tgataaattt taaccgtatg aatacctatg caaccagagg    300
   gtacaggcca cattaccccc acttaatcca ctgaagctgc catttttcat ggtttcacca    360
   tcccagcgaa gggccatcca gcgtgcgttc ctgtatttcc gactggcgcg ccattcaggt    420
   ggcacttttc ggggaaatgt gcgcggaacc cctatttgtt tatttttcta aatacattca    480
   aatatgtatc cgctcatgaa ttaattctta gaaaaactca tcgagcatca aatgaaactg    540
   caatttattc atatcaggat tatcaatacc atatttttga aaaagccgtt tctgtaatga    600
   aggagaaaac tcaccgaggc agttccatag gatggcaaga tcctggtatc ggtctgcgat    660
   tccgactcgt ccaacatcaa tacaacctat taatttcccc tcgtcaaaaa taaggttatc    720
   aagtgagaaa tcaccatgag tgacgactga atccggtgag aatggcaaaa gtttatgcat    780
   ttctttccag acttgttcaa caggccagcc attacgctcg tcatcaaaat cactcgcatc    840
   aaccaaaccg ttattcattc gtgattgcgc ctgagcgaga cgaaatacgc gatcgctgtt    900
   aaaaggacaa ttacaaacag gaatcgaatg caaccggcgc aggaacactg ccagcgcatc    960
   aacaatattt tcacctgaat caggatattc ttctaatacc tggaatgctg ttttcccggg   1020
   gatcgcagtg gtgagtaacc atgcatcatc aggagtacgg ataaaatgct tgatggtcgg   1080
   aagaggcata aattccgtca gccagtttag tctgaccatc tcatctgtaa catcattggc   1140
   aacgctacct ttgccatgtt tcagaaacaa ctctggcgca tcgggcttcc catacaatcg   1200
   atagattgtc gcacctgatt gcccgacatt atcgcgagcc catttatacc catataaatc   1260
   agcatccatg ttggaattta atcgcggcct agagcaagac gtttcccgtt gaatatggct   1320
   cataacaccc cttgtattac tgtttatgta agcagacagt tttattgttc atgaccaaaa   1380
   tcccttaacg tgagttttcg ttccactgag cgtcagaccc cgtagaaaag atcaaaggat   1440
   cttcttgaga tccttttttt ctgcgcgtaa tctgctgctt gcaaacaaaa aaaccaccgc   1500
   taccagcggt ggtttgtttg ccggatcaag agctaccaac tctttttccg aaggtaactg   1560
   gcttcagcag agcgcagata ccaaatactg tccttctagt gtagccgtag ttaggccacc   1620
   acttcaagaa ctctgtagca ccgcctacat acctcgctct gctaatcctg ttaccagtgg   1680
   ctgctgccag tggcgataag tcgtgtctta ccgggttgga ctcaagacga tagttaccgg   1740
   ataaggcgca gcggtcgggc tgaacggggg gttcgtgcac acagcccagc ttggagcgaa   1800
   cgacctacac cgaactgaga tacctacagc gtgagctatg agaaagcgcc acgcttcccg   1860
   aagggagaaa ggcggacagg tatccggtaa gcggcagggt cggaacagga gagcgcacga   1920
   gggagcttcc agggggaaac gcctggtatc tttatagtcc tgtcgggttt cgccacctct   1980
   gacttgagcg tcgatttttg tgatgctcgt caggggggcg gagcctatgg aaaaacgcca   2040
   gcaacgcggc ctttttacgg ttcctggcct tttgctggcc ttttgctcac atgttctttc   2100
   ctgcgttatc ccctgattct gtgggtaacc gtattaccgc ctttgagtga gctgataccg   2160
   ctcgccgcag ccgaacgacc gagcgcagcg agtcagtgag cgaggaagcg gaagagcgcc   2220
   tgatgcggta ttttctcctt acgcatctgt gcggtatttc acaccgcaat ggtgcactct 2280
   cagtacaatc tgctctgatg ccgcatagtt aagccagtat acactccgct atcgctacgt 2340
   gactgggtca tggctgcgcc ccgacacccg ccaacacccg ctgacgcgcc ctgacgggct 2400
   tgtctgctcc cggcatccgc ttacagacaa gctgtgaccg tctccgggag ctgcatgtgt 2460
   cagaggtttt caccgtcatc accgaaacgc gcgaggcagc tgcggtaaag ctcatcagcg 2520
   tggtcgtgaa gcgattcaca gatgtctgcc tgttcatccg cgtccagctc gttgagtttc 2580
   tccagaagcg ttaatgtctg gcttctgata aagcgggcca tgttaagggc ggttttttcc 2640
   tgtttggtca ctgatgcctc cgtgtaaggg ggatttctgt tcatgggggt aatgataccg 2700
   atgaaacgag agaggatgct cacgatacgg gttactgatg atgaacatgc ccggttactg 2760
   gaacgttgtg agggtaaaca actggcggta tggatgcggc gggaccagag aaaaatcact 2820
   cagggtcaat gccagcgctt cgttaataca gatgtaggtg ttccacaggg tagccagcag 2880
   catcctgcga tgcagatccg gaacataatg gtgcagggcg ctgacttccg cgtttccaga 2940
   ctttacgaaa cacggaaacc gaagaccatt catgttgttg ctcaggtcgc agacgttttg 3000
   cagcagcagt cgcttcacgt tcgctcgcgt atcggtgatt cattctgcta accagtaagg 3060
   caaccccgcc agcctagccg ggtcctcaac gacaggagca cgatcatgcg cacccgtggg 3120
   gccgccatgc cggcgataat ggcctgcttc tcgccgaaac gtttggtggc gggaccagtg 3180
   acgaaggctt gagcgagggc gtgcaagatt ccgaataccg caagcgacag gccgatcatc 3240
   gtcgcgctcc agcgaaagcg gtcctcgccg aaaatgaccc agagcgctgc cggcacctgt 3300
   cctacgagtt gcatgataaa gaagacagtc ataagtgcgg cgacgatagt catgccccgc 3360
   gcccaccgga aggagctgac tgggttgaag gctctcaagg gcatcggtcg agatcccggt 3420
   gcctaatgag tgagctaact tacattaatt gcgttgcgct cactgcccgc tttccagtcg 3480
   ggaaacctgt cgtgccagct gcattaatga atcggccaac gcgcggggag aggcggtttg 3540
   cgtattgggc gccagggtgg tttttctttt caccagtgag acgggcaaca gctgattgcc 3600
   cttcaccgcc tggccctgag agagttgcag caagcggtcc acgctggttt gccccagcag 3660
   gcgaaaatcc tgtttgatgg tggttaacgg cgggatataa catgagctgt cttcggtatc 3720
   gtcgtatccc actaccgaga tatccgcacc aacgcgcagc ccggactcgg taatggcgcg 3780
   cattgcgccc agcgccatct gatcgttggc aaccagcatc gcagtgggaa cgatgccctc 3840
   attcagcatt tgcatggttt gttgaaaacc ggacatggca ctccagtcgc cttcccgttc 3900
   cgctatcggc tgaatttgat tgcgagtgag atatttatgc cagccagcca gacgcagacg 3960
   cgccgagaca gaacttaatg ggcccgctaa cagcgcgatt tgctggtgac ccaatgcgac 4020
   cagatgctcc acgcccagtc gcgtaccgtc ttcatgggag aaaataatac tgttgatggg 4080
   tgtctggtca gagacatcaa gaaataacgc cggaacatta gtgcaggcag cttccacagc 4140
   aatggcatcc tggtcatcca gcggatagtt aatgatcagc ccactgacgc gttgcgcgag 4200
   aagattgtgc accgccgctt tacaggcttc gacgccgctt cgttctacca tcgacaccac 4260
   cacgctggca cccagttgat cggcgcgaga tttaatcgcc gcgacaattt gcgacggcgc 4320
   gtgcagggcc agactggagg tggcaacgcc aatcagcaac gactgtttgc ccgccagttg 4380
   ttgtgccacg cggttgggaa tgtaattcag ctccgccatc gccgcttcca ctttttcccg 4440
   cgttttcgca gaaacgtggc tggcctggtt caccacgcgg gaaacggtct gataagagac 4500
   accggcatac tctgcgacat cgtataacgt tactggtttc acattcacca ccctgaattg 4560
   actctcttcc gggcgctatc atgccatacc gcgaaaggtt ttgcgccatt cgatggtgtc 4620
   cgggatctcg acgctctccc ttatgcgact cctgcattag gaagcagccc agtagtaggt 4680
   tgaggccgtt gagcaccgcc gccgcaagga atggtgcatg caaggagatg gcgcccaaca 4740
   gtcccccggc cacggggcct gccaccatac ccacgccgaa acaagcgctc atgagcccga 4800
   agtggcgagc ccgatcttcc ccatcggtga tgtcggcgat ataggcgcca gcaaccgcac 4860
   ctgtggcgcc ggtgatgccg gccacgatgc gtccggcgta gaggatcgag atctcgatcc 4920
   cgcgaaatta atacgactca ctatagggga attgtgagcg gataacaatt cccctctaga 4980
   aataattttg tttaacttta agaaggagat ataccatggg cagcagccat catcatcatc 5040
   atcacagcag cggcctggtg ccgcgcggca gccatatggc tagcatgact ggtggacagc 5100
   aaatgggtcg gatccgaatt cgagctccgt cgacaagctt gcggccgcac tcgagcacca 5160
   ccaccaccac cactgagatc cggctgctaa caaagcccga aaggaagctg agttggctgc 5220
   tgccaccgct gagcaataac tagcataacc ccttggggcc tctaaacggg tcttgagggg 5280
   ttttttgctg aaaggaggaa ctatatccgg at 5312

## Description

La présente invention se rapporte à un antigène polypeptidique dérivant de la protéine gp41, ainsi qu'à son utilisation pour l'immunisation contre les infections liées au VIH., ces travaux ont été co-financés par l'ANRS.

La mise au point d'une méthode d'immunisation contre le VIH est aujourd'hui l'une des priorités de la recherche scientifique.

Les obstacles majeurs que sont la grande variabilité génétique du virus et la faible exposition au système immunitaire d'épitopes viraux neutralisants freinent considérablement l'élaboration d'une immunité neutralisante.

La glycoprotéine d'enveloppe du VIH qui est requise pour conférer au virus son caractère infectieux représente la cible d'anticorps neutralisants. Ces caractéristiques ont fait de cette dernière un sujet d'investigations intenses.

L'enveloppe glycoprotéique (env), du virus d'immunodéficience humaine-1 (VIH-1) est synthétisée à partir du précurseur gp160 qui donne sous l'action d'une protéase les sous-unités gp120 et gp41.

La fixation de gp120/gp41 aux récepteurs cellulaires induit un changement de conformation de la gp41 d'un état latent (non-fusogène) à un état fusion-actif (fusogène). Entre ces deux états, existe un état transitoire dit "intermédiaire" pendant lequel la gp41 se présente comme une protéine membranaire à la fois dans les membranes virale et cellulaire (Weissenhorn et al Nature (1997), 387 (6631), 426-30).

L'utilisation à des fins vaccinales de la gp41 dans sa conformation fusogénique est décrite dans WO00/40616. Selon cette demande, les hélices N peuvent être utilisées seules ou en association avec les hélices C pour reproduire dans ce dernier cas la conformation fusogène de la gp41.

Des expériences de liaison ont permis d'établir d'autre part que l'état latent non-fusogène est caractérisé par l'inaccessibilité de larges portions de l'ectodomaine de la gp41. La gp120 interagit en effet de sorte à masquer les épitopes. Il a par ailleurs été montré que l'inhibition du changement de structure de l'état intermédiaire vers l'état fusogène par des peptides utilisés en tant que compétiteurs pouvait affecter l'infection virale (Weissenhorn W. et al, Molecular Membrane Biology, 1999, 16, 3-9).

La demanderesse propose un nouvel antigène polypeptidique utilisable pour l'immunisation thérapeutique et prophylactique contre les infections liées au VIH. La demanderesse a en effet mis en évidence pour la première fois qu'un polypeptide mimant l'état intermédiaire de la gp41 est capable d'induire des anticorps neutralisant les isolats primaires du VIH.

La présente invention concerne donc un polypeptide comprenant une séquence de formule I:

[N-S1]n-C-[S2-N]m

dans laquelle :
N représente la séquence des acides aminés 30 à 77 de la gp41,
C représente la séquence des acides aminés 117 à 154 de la gp41,
S1 et S2 sont indépendamment l'un de l'autre soit absents, soit représentent une séquence d'acides aminés telle que la séquence de formule I adopte une conformation en hélice alpha telle que déterminée par le logiciel SOPMA dans les conditions suivantes :
- nombre d'états conformationnels = 4
- Limite de similarité = 8 et,
- Largeur de la fenêtre = 70
n = 0 ou 1 ; m = 0 ou 1 et m + n = 1 ou 2.

De préférence, le polypeptide tel que défini ci-dessus comprend une séquence de formule I dans laquelle m + n = 1.

De préférence S1 est absent ou représente la séquence d'acides aminés D, DQ, DQQ, DQQL ou DNNMT et S2 est absent ou représente la séquence d'acides aminés W, WA, WAS, WASL ou WASLW.

Les séquence d'acides aminés S1 et S2 sont définies en utilisant le code à une lettre dans lequel D représente l'acide aspartique, Q représente la glutamine, etc...

Selon un mode de réalisation particulier le polypeptide comprend une séquence de formule I telle que définie ci-dessus dans laquelle N représente SEQ ID N°26 et C représente SEQ ID N°27.

Selon un autre mode de réalisation préféré, le polypeptide selon l'invention est sélectionné dans le groupe consistant en SEQ ID N° 28 et SEQ ID N°31.

Selon un autre mode de réalisation, le polypeptide selon l'invention comprend une séquence supplémentaire de formule (G)a-S-(H)b dans laquelle G représente un résidu glycine, H représente un résidu histidine, S étant un résidu sérine, a est supérieur ou égal à 4 et b est supérieur ou égal à 6. Ladite séquence est liée par liaison amide à l'extrémité NH2-terminale ou COOH-terminale du polypeptide selon l'invention.

Selon un autre aspect la présente invention concerne un conjugué comprenant un polypeptide selon l'invention conjugué à une protéine ou un peptide porteur.

Selon un autre aspect, la présente invention concerne une séquence d'ADN codant pour un polypeptide selon l'invention ou pour un conjugué selon l'invention.

La présente invention concerne également un vecteur d'expression comprenant ladite séquence d'ADN ainsi qu'une cellule hôte contenant ledit vecteur.

La présente invention a également pour objet une composition pharmaceutique comprenant au moins un polypeptide tel que défini ci-dessus, ou au moins un conjugué tel que défini ci-dessus, ou au moins un vecteur d'expression tel que défini ci-dessus, un excipient pharmaceutiquement acceptable et éventuellement un adjuvant.

Selon un mode de réalisation particulier, la présente invention concerne une composition pharmaceutique telle que définie ci-dessus administrable par voie orale.

La présente invention a donc également pour objet le polypeptide tel que défini ci-dessus pour son utilisation à titre de médicament et en particulier pour son utilisation pour l'immunisation du corps humain contre les infections liées au VIH.

Un autre objet de la présente invention a trait au procédé de préparation d'un polypeptide tel que défini ci-dessus comprenant l'expression dudit polypeptide à partir d'une cellule hôte telle que définie ci-dessus.

L'invention est décrite plus en détails dans la description qui suit.

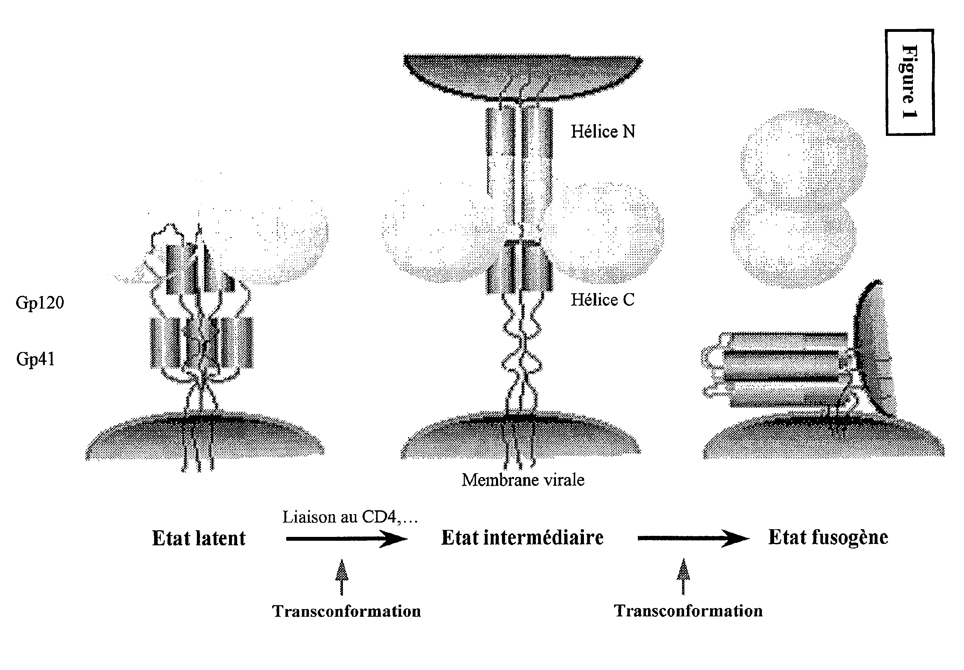

Le phénomène de changement de conformation de la gp41 précédant la fusion des membranes cellulaires et virales est illustré à la figure 1. La fixation de la gp120 sur le récepteur et le co-récepteur du virus provoque une première modification qui entraîne le dépliement du peptide de fusion et son ancrage dans la membrane cellulaire. A ce moment un état intermédiaire se forme dans lequel il n'y a pas d'interaction entre les hélices N et C. Le second événement illustré à la figure 1 est le repliement de la gp41 qui correspond à l'adoption d'une conformation thermodynamiquement plus stable de la molécule. L'énergie libérée au cours de ce repliement permet le rapprochement et la fusion des lipides des membranes cellulaire et virale.

La demanderesse a mis en évidence de façon surprenante que le polypeptide selon l'invention induisait des anticorps IgG spécifiques neutralisant les isolats primaires du VIH. L'induction d'anticorps neutralisant les isolats primaires peut être déterminée par le test de neutralisation tel que décrit dans l'article de C. Moog et al (AIDS Research and human retroviruses, vol. 13(1), 13-27, 1997) auquel on pourra se référer pour un descriptif complet de ce dernier. On estime dans le cadre de la présente invention que des anticorps neutralisants ont été induits par l'antigène testé selon la technique de C. Moog lorsque le sérum dilué au moins au 1/4 en présence de VIH entraîne une réduction d'un facteur 10 du titre viral en comparaison au VIH seul, le titre viral étant évalué par la quantité de p24 produite dans le surnageant de culture.

L'induction d'anticorps neutralisant les isolats primaires peut également être déterminée par le test de neutralisation de D. Montefiori tel que décrit dans J. Infect. Dis. 1996, 173:60-67. Dans ce test, le titre neutralisant est exprimé par le pourcentage de réduction d'antigène p24 produit dans les surnageants de culture lorsque le virus est incubé en présence de sérum dilué au ¼ par comparaison au virus en absence de sérum. On considère dans le cadre de la présente invention que des anticorps neutralisants ont été induits lorsque la réduction du taux de p24 produit atteint au moins 80% avec un sérum dilué au ¼. On considère dans le cadre de la présente invention que les anticorps induits par le polypeptide selon l'invention sont neutralisants si une activité neutralisante est détectée pour un isolat donné dans au moins un des deux tests ci-dessus.

L'induction d'anticorps neutralisant la souche VIH-1 de laboratoire MN peut être estimée en utilisant la lignée cellulaire MT-2 selon la méthode de D. Montefiori, décrite dans : DC Montefiori *et al.* J Clin Microbiol. 1988, 26: 231-5). Dans cette méthode, le titre neutralisant est exprimé comme l'inverse de la dilution du sérum (en valeur arithmétique) protégeant au moins 50% de cellules de l'effet cytopathogène du virus VIH.

Cette propriété fait du polypeptide selon l'invention un candidat vaccinal d'intérêt pour l'homme.

Le polypeptide selon la présente invention à la particularité d'adopter, dans des conditions physiologiques, une conformation que l'on peut qualifier « d'ouverte » par opposition à la conformation « fermée » de la gp41 sous forme fusogène dans laquelle les domaines N et C sont appariés les uns aux autres selon une orientation anti-parallèle (Cf. Fig. 1). En effet, la conformation ouverte du polypeptide selon l'invention se caractérise par le fait que la séquence C n'est pas appariée à la séquence N selon l'orientation anti-parallèle telle que présente dans la forme fusogène. Le polypeptide selon la présente invention est de préférence sous une forme trimérique dans laquelle les séquences N et les séquences C sont respectivement appariées entre elles selon une orientation parallèle.

La demanderesse a également mis en évidence de façon surprenante que le polypeptide selon l'invention conservait sa conformation « ouverte » en milieu fortement acide. Cette propriété fait du polypeptide selon l'invention un antigène vaccinal administrable par voie orale. La demanderesse a en effet montré que l'ectodomaine de la protéine gp41 est extrêmement thermostable à pH2,5. La mesure du Tm (température à laquelle 50% des protéines présentes sont dénaturées) de la gp41 dans 50mM de formate de sodium par DSC (calorimétrie différentielle à balayage) donne une valeur de 110°C, le début du phénomène de dénaturation apparaissant à environ 100°C à pH=2,5. La thermostabilité de la protéine gp41 à pH neutre a été évaluée par Weissenhorn et al (EMBO, 1996, 7, 1507-1514). Le Tm mesuré à pH neutre par ces auteurs est de 78°C, ce qui signifie que de façon surprenante cette protéine est plus stable à pH acide qu'à pH neutre. Ces résultats ont été confirmé par une analyse par dichroïsme circulaire visant à calculer le pourcentage d'hélice alpha dans la protéine. La demanderesse a également montré que le polypeptide selon l'invention présentait la même particularité. Cette propriété spécifique fait du polypeptide selon l'invention un antigène vaccinal de choix pour une administration par voie orale.

Cette conformation ouverte est obtenue par la liaison directe des séquences N et C telles que définies ci-dessus. Dans un tel cas, S1 et S2 sont absents et le dernier acide aminé de la séquence N est directement lié par liaison amide au premier acide aminé de la séquence C ou inversement (ex : AA33-77-AA117-154). Cette conformation ouverte est également obtenue lorsque les séquences N et C telles que définies ci-dessus sont reliées entre elles via une séquence S1 ou S2 telle que la séquence résultante de formule I adopte une conformation en hélice alpha telle que déterminée par le logiciel SOPMA dans les conditions suivantes : nombre d'états conformationnels = 4 ; limite de similarité = 8 et largeur de la fenêtre = 70.

La conformation ouverte selon l'invention se caractérise en effet par l'absence d'une région flexible entre les séquences N et C. Le logiciel SOPMA disponible sur le site : http://npsa-pbil.ibcp.fr/cgi-bin/npsa_automat.pl ?page=/NPSA/npsa_sopma.html (Geourjon C et Deléage G. SOPMA, Casios (1995), 11, 681-684) permet de déterminer aisément si la séquence S1 ou S2 adopte dans le cadre du polypeptide de formule I une conformation en hélice alpha appropriée dans le cadre de la présente invention.

Selon un mode de réalisation préféré m + n = 1 et si S1 est absent S2 représente W, WA, WAS, WASL ou WASLW et si S2 est absent S1 représente D, DQ, DQQ, DQQL ou DNNMT.

De plus, selon un mode de réalisation particulièrement préféré, dans le polypeptide tel que défini ci-dessus les séquences AA30-AA77 et AA117-AA154 correspondent respectivement aux séquences SEQ ID N°26 et SEQ ID N°27.

Selon un mode de réalisation préféré, le polypeptide selon l'invention est sélectionné dans le groupe consistant en SEQ ID N°28 et SEQ ID N°31.

Les séquences AA30-AA77 et AA117-AA154 issues de la gp41 sont numérotées en prenant pour référence la numérotation de la séquence de la protéine gp41 telle que donnée dans le listing de séquences sous l'intitulé SEQ ID N°25. La séquence SEQ ID N°25 correspond une protéine gp41 LAI tronquée dans laquelle le premier acide aminé A porte le numéro 1.

Les séquences AA30-AA77 et AA117-AA154 selon la présente invention peuvent être issues de toute protéine gp 41 du VIH, incluant les souches VIH1 et VIH2, incluant les souches de laboratoire et les isolats primaires. De préférence les séquences N et C selon la présente invention sont issues d'une souche VIH1 et en particulier d'une souche VIH 1 LAI.

Les séquences nucléotidiques et peptidiques d'un grand nombre de protéine gp41 sont connues et disponibles par exemple sur Internet sur le site http://hiv-web.lanl.gov/ ainsi que dans les compendium correspondant de Los Alamos. Il est clair que toute séquence dans laquelle une ou plusieurs mutations conservatives qui n'altèrent substantiellement ni l'immunogénicité ni la conformation ouverte ont été introduites est également incluse dans le cadre de la présente invention.

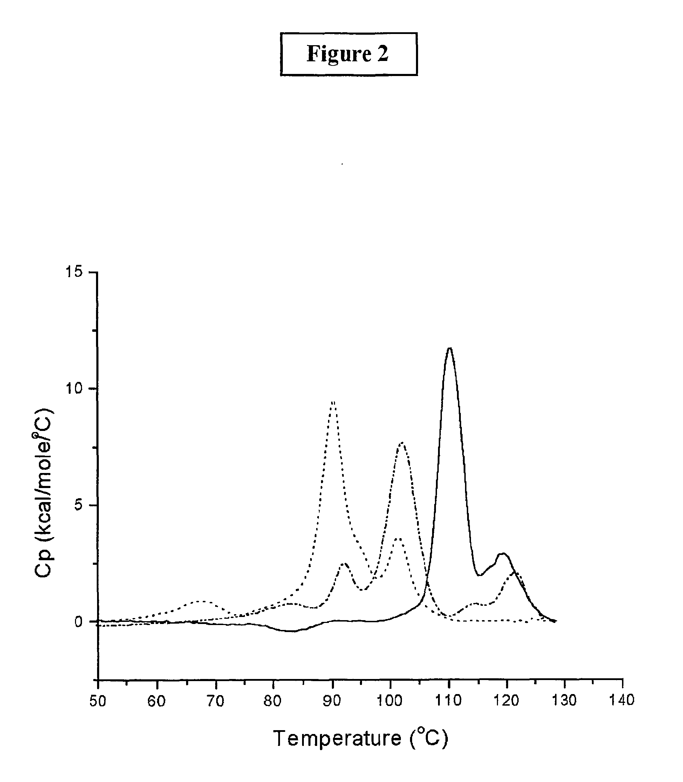

La conformation ouverte selon la présente invention se caractérise par un certain nombre de paramètres mesurables par la technique de DSC et la technique de dichroïsme circulaire. Ces techniques sont décrites en détails dans les articles suivants de A. Cooper et al, Phil Trans. R. Soc. Lon. A (1993) 345, 23-25, et de V.V. Plotnikov et al Analytical Biochemistry 250, 237-244, (1997) et S.M. Kelly et N.C. Price (1997) Biochim. Biophys. Acta. 1338,161-185. La DSC mesure les paramètres thermodynamiques de la dénaturation des protéines. La dénaturation des protéines est un événement endothermique évaluable par la mesure de l'absorption de la chaleur par la protéine en fonction de la température. Les deux principaux paramètres obtenus sont :
- le Tm ou point de demi-dénaturation (i.e. température à laquelle 50% de la protéine est présente sous forme native et 50% sous forme dénaturée) et
- le ΔH ou variation d'enthalpie pendant la dénaturation (i.e. la chaleur nécessaire pour dénaturer la protéine).
Ces deux paramètres sont des marqueurs précis de la conformation d'une protéine.
Une analyse par DSC est détaillée dans l'exemple 5 par référence à la figure 2.

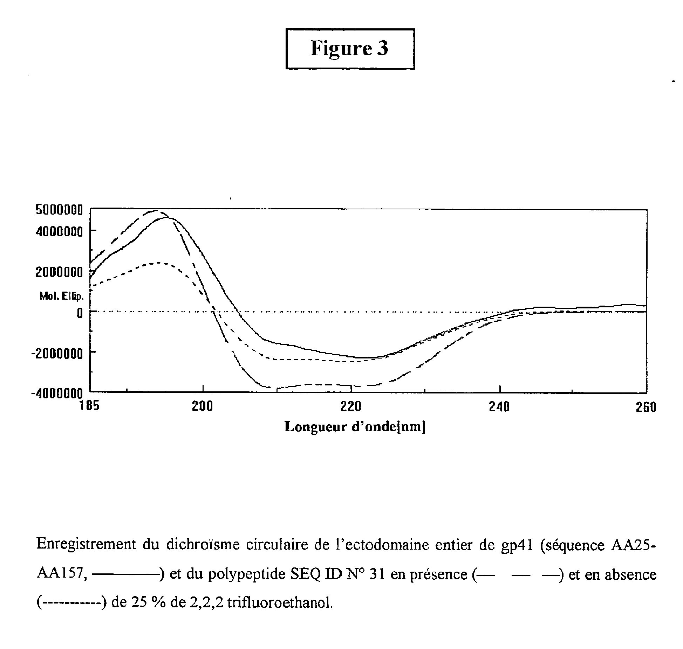

La technique de dichroïsme circulaire permet quant à elle d'évaluer la structure secondaire de la protéine. Le signal d'une hélice alpha est caractérisé par des minima à 208nm et 222nm. La valeur du signal à 222nm est utilisée pour déterminer le pourcentage d'hélice alpha dans une protéine. Le signal d'une boucle est caractérisé par un maximum à environ 205nm. Cette technique permet de mettre en évidence que le polypeptide selon l'invention ne contient pas la boucle normalement présente dans la forme fusogène et qu'il est constitué presque exclusivement, voire exclusivement, d'une hélice alpha.
Une analyse par dichroïsme circulaire est détaillée dans l'exemple 6 par référence à la figure 3.

Bien que le polypeptide selon la présente invention présente une conformation ouverte qui soit stable, cette conformation peut être renforcée par addition de résidus cystéines aux extrémités du polypeptide. A cette fin, deux résidus cystéine supplémentaires peuvent être ajoutés en N-terminal ou en C-terminal, de préférence en N-terminal du polypeptide selon l'invention pour fixer par covalence le trimère dans une conformation ouverte.

Le polypeptide selon l'invention peut être obtenu par toute technique classique de synthèse chimique ou de génie génétique. Lorsque le polypeptide est produit par synthèse chimique, le polypeptide selon l'invention peut être synthétisé sous la forme d'une séquence unique, ou sous la forme de plusieurs séquences qui sont ensuite liées les unes aux autres. La synthèse chimique peut être réalisée en phase solide ou en solution, ces deux techniques de synthèse étant bien connues de l'homme de l'art. Ces techniques sont décrites notamment par Atherton et Shepard dans « solid phase peptide synthesis (IRL press Oxford, 1989) et par Houbenweyl dans « method der organischen chemie » édité par E.Wunsch vol, 15-I et II thieme, Stuttgart, 1974, ainsi que dans les articles suivants dont l'intégralité est incorporée ici à titre de référence : Dawson PE et al (Synthesis of proteins by native chemical ligation Science 1994 ; 266(5186):776-9);Kochendoerfer GG et al (Chemical protein synthesis. Curr Opin Chem Biol 1999 ;3(6):665-71); et Dawson PE et al Synthesis of native proteins by chemical ligation,Annu Rev Biochem 2000 ; 69:923-60.

Le polypeptide selon l'invention peut être également produit par les techniques de génie génétique bien connues de l'homme de l'art. Lorsque le polypeptide selon l'invention est produit par génie génétique il comprend à l'extrémité NH₂-terminale de la séquence de formule I un résidu méthionine supplémentaire correspondant à la traduction du premier codon d'initiation et peut comprendre également quelques acides aminés supplémentaire en N et C-terminal. Ces techniques sont décrites en détails dans Molecular Cloning : a molecular manual de Maniatis et al, Cold Spring Harbor, 1989). Classiquement, la séquence d'ADN codant pour le polypeptide selon l'invention peut être produite par la technique de PCR dans laquelle les séquences N et C sont dans un premier temps amplifiées indépendamment l'une de l'autre puis sont dans un deuxième temps appariées et de nouveau amplifiées. La séquence d'ADN ainsi obtenue est ensuite insérée dans un vecteur d'expression. Le vecteur d'expression renfermant la séquence d'intérêt est alors utilisé pour transformer une cellule hôte permettant l'expression de la séquence d'intérêt. Le polypeptide produit est ensuite isolé du milieu de culture par des techniques classiques bien connues de l'homme de l'art, telles que précipitation à l'éthanol ou au sulfate d'ammonium, extraction par l'acide, chromatographie d'échange d'anion/cation, chromatographie sur phosphocellulose, chromatographie d'interaction hydrophobe, chromatographie d'affinité, chromatographie sur hydroxyapatite et chromatographie sur lectine. De préférence, la chromatographie liquide à haute performance (HPLC) est utilisée dans la purification.
Selon le système expression utilisé (protéine sécrétée ou non) et selon le procédé de purification, le polypeptide purifié peut se présenter sous différentes formes. Il peut être sous une forme dénaturée ou non dénaturée, monomérique ou multimérique. Lorsqu'il se trouve sous une forme dénaturée il est possible de lui rendre sa conformation ouverte selon l'invention en utilisant le procédé décrit dans les exemples donnés ci-après. Pour obtenir des formes multimériques et en particulier des trimères les molécules de polypeptide purifiées doivent être placées dans un milieu qui permet aux molécules d'être parfaitement solubles et essentiellement sans interaction entre elles et de préférence sans structure secondaire. Pour cela on peut utiliser des détergents tels que dodecyl sulfate de sodium, N lauryl sarcosine, Guanidinium chloride, Urée, Thiocyanate de sodium ou des agents chaotropiques. On pourra favoriser les conditions désirées par l'utilisation de solvants organiques ou l'utilisation d'acides. Cette première condition étant satisfaite, on place l'échantillon dans une cassette de dialyse pour éliminer une partie des détergents ou des agents chaotropiques utilisés, de façon à favoriser les interactions entre les monomères de polypeptide en conservant aux molécules suffisamment de solubilité. Dans une deuxième étape, la formation des trimères étant favorisée, on dialyse complètement l'échantillon dans un milieu physiologique qui maintient le polypeptide en solution ou en suspension. On obtient alors des trimères du polypeptide selon l'invention. Une telle technique est décrite en détail dans la demande WO00/08167.

Pour mettre en oeuvre la synthèse du polypeptide, tout vecteur d'expression classiquement utilisé pour l'expression d'une protéine recombinante peut être utilisé dans le cadre de la présente invention. Ce terme englobe donc aussi bien les vecteurs d'expression dits « vivants » tels que les virus et les bactéries que les vecteurs d'expression de type plasmidiques.

On utilise de préférence des vecteurs dans lesquels la séquence d'ADN du polypeptide selon l'invention est sous la dépendance d'un promoteur fort, inductible ou non inductible. On peut citer à titre d'exemple de promoteur utilisable, le promoteur de l'ARN polymérase T7.

Les vecteurs d'expression incluent de préférence au moins un marqueur de sélection. De tels marqueurs incluent, par exemple, le gène de la dihydrofolate réductase ou le gène de résistance à la néomycine pour la culture des cellules d'eucaryote et les gènes de résistance à la kanamycine, tétracycline ou ampicilline pour la culture dans *E. coli* et des autres bactéries.

On peut citer à titre de vecteur d'expression utilisable dans le cadre de la présente invention les plasmides pET28 (Novagen) ou pBAD (Invitrogen) par exemple ; les vecteurs viraux tels que : baculovirus, poxvirus en particulier les poxvirus décrits dans les brevets US 5,942,235, US 5,756,103 et US 5,990,091 dont l'intégralité est incorporée ici à titre de référence, les virus de la vaccine recombinants, en particulier les virus recombinants décrits dans les brevets EP 83286, US 5,494,807 et US 5,762,938 dans lesquels est cloné la séquence d'ADN codant pour un polypeptide selon l'invention.

Pour favoriser l'expression et la purification du polypeptide, ce dernier peut être exprimé dans une forme modifiée, telle qu'une protéine de fusion, et peut inclure non seulement des signaux de sécrétion, mais aussi des régions fonctionnelles hétérologues supplémentaires. Par exemple, une région d'acides aminés supplémentaires, particulièrement des acides aminés chargés, peut être ajoutée au N-terminal du polypeptide pour améliorer la stabilité et le maintien dans la cellule hôte.

Pour l'expression du polypeptide, toute cellule hôte classiquement utilisée en association avec les vecteurs d'expression décrits ci-dessus peut être utilisée.

On peut citer à titre d'exemple non limitatif les cellules de E. coli, BL21 (λDE3), HB101, Top 10, CAG 1139, Bacillus, les cellules eucaryotes telles que CHO ou Vero.

De préférence on utilisera dans le cadre de la présente invention le système vecteur d'expression /cellule suivant : pET(Cer) /BL21LamdaDE3, ou BL21lamdaDE3(RIL).

En fonction de la cellule hôte utilisée pour l'expression du polypeptide , les polypeptides de la présente invention pourront être glycosylés ou non-glycosylés. De plus, les polypeptides selon l'invention pourront aussi inclure un résidu de méthionine supplémentaire en N-terminal. Des acides aminés supplémentaires en N et C-terminal peuvent également résulter du processus de recombinaison.
Une fois purifié, le polypeptide selon l'invention peut avantageusement être mélangé avec du 2,2,2-trifluoroéthanol (TFE). Pour ce faire, le polypeptide est de préférence mis dans un tampon acide tel que formate de sodium à un pH=2,5. Le mélange ainsi formé contient de 10 à 50% en vol de TFE, de préférence de 15 à 30% en vol de TFE. Le TFE a pour effet d'augmenter le taux d'hélicité du polypeptide pour le porter à une valeur proche ou égale à 100%.
Outre le TFE, d'autres solvants organiques ou détergents peuvent également être utilisés. On peut citer à titre d'exemple : l'isopropanol ou le lyso phospholipide. Les quantités appropriées de ces composés peuvent être aisément déterminées par l'homme de l'art.
Ces composés étant en général toxiques et par voie de conséquence non administrables chez l'homme, il est nécessaire de les éliminer avant administration. La demanderesse a mis en évidence que ces solvants ou détergents pouvaient être aisément éliminés par l'ajout d'un support adsorbant le polypeptide. Le support utilisé dans le cadre de la présente invention correspond à un support pharmaceutiquement acceptable pouvant être administré chez l'homme. On peut citer à titre d'exemple de support utilisable dans le cadre de la présente invention les gels d'hydroxyde, de phosphate, ou hydroxy-phosphate d'aluminium qui sont classiquement utilisés comme adjuvant dans les vaccins. Le support est utilisé en large excès par rapport au polypeptide pour obtenir une adsorption totale de ce dernier.
Tout autre support pharmaceutiquement acceptable et susceptible d'adsorber le polypeptide selon l'invention pourrait être utilisé dans le cadre de la présente invention.

Classiquement la quantité requise de support pharmaceutiquement acceptable est ajouté au mélange polypeptide/TFE puis l'ensemble est incubé à température ambiante pendant la durée nécessaire pour permettre l'absorption totale du polypeptide sur le support. La durée d'incubation peut varier de 15 min. à 3H. le mélange est ensuite centrifugé 1 ou 2 fois à environ 10000g et le culot est repris dans une solution administrable chez l'homme telle que par exemple dans le cas d'une composition injectable du tampon PBS ou une solution saline physiologique.

La présente invention a également pour objet les conjugués comprenant un polypeptide selon l'invention et une protéine porteur ou un peptide porteur.

La protéine (ou peptide) porteur renforce l'immunogénicité du polypeptide selon l'invention notamment en augmentant la production d'anticorps spécifiques. Ladite protéine (ou peptide) porteur comprend de préférence un ou plusieurs épitope(s) T helper. Par « épitope T helper », on entend un enchaînement d'acides aminés qui, dans le contexte d'une ou de plusieurs molécules du MHC classe II, active les lymphocytes T helper. Selon un mode de réalisation avantageux, la protéine (ou peptide) porteur utilisée améliore la solubilité dans l'eau du polypeptide selon l'invention.

Comme protéine porteur on peut utiliser par exemple les protéines de surface de phages comme la protéine pIII ou pVIII du phage M13, les protéines de surface bactériennes comme les protéines LamB, OmpC, ompA, ompF et PhoE d'E. coli, la protéine CotC ou CotD de B. Subtilis, les porines bactériennes comme la porine P1 de Neisseria gonorrheae, la porine P1
ou P2 d'H. influenzae B, la porine de classe 1 de N. meningitidis B, la porine P40 de K. pneumoniae, des lipoprotéines telles que OspA de B. bugdorfi, PspA de S. pneumoniae, TBP2 de N. meningitidis B, TraT d'E. coli ainsi que l'adhésine A de S. pneumoniae ; les « heat shock » protéines telles que Hsp65 ou Hsp71 de M. tuberculosis ou bovis ou Hin 47 d'H. infuenzae type B. Sont également particulièrement appropriées dans le cadre de la présente invention, les toxines bactériennes détoxifiées comme l'anatoxine tétanique ou diphtérique, la sous unité B de la toxine cholérique, la sous unité B de la toxine cholérique, la sous unité B de l'endotoxine A de P. aeruginosa ou l'exotoxine A de S. aureus.

Comme peptide porteur on peut utiliser par exemple dans le cadre de la présente invention les peptides p24E, p24N, p24H et p24M décrits dans WO94/29339 ainsi que les peptides PADRE tels que décrits par Del guercio et al (Vaccine (1997) ; vol15/4, p441-448).

La protéine (ou peptide) porteur est liée à l'extrémité N ou C terminale du polypeptide selon l'invention par tout procédé de conjugaison bien connu de l'homme de l'art. De plus, la séquence codant pour la protéine (ou peptide) porteur peut avantageusement être fusionnée à la séquence codant pour le polypeptide selon l'invention et la séquence résultante peut être exprimée sous la forme d'une protéine de fusion par tout procédé classique. Toutes les techniques de génie génétique utiles pour ce faire sont décrites dans le Maniatis et al. Lesdits conjugués peuvent être isolés par tout procédé classique de purification bien connus de l'homme de l'art.

La présente invention a également pour objet les séquences d'ADN codant pour les polypeptides et les conjugués selon l'invention ainsi que les vecteurs d'expression comprenant lesdites séquences et les cellules hôtes transformées par lesdits vecteurs. Les séquences d'ADN codant pour les polypeptides selon l'invention peuvent être aisément produite par PCR en utilisant comme matrice la séquence nucléotidique d'une protéine gp41.

Plutôt que d'extraire et de purifier le polypeptide ou le conjugué exprimé par le vecteur d'expression il est souvent plus facile et parfois plus avantageux d'utiliser le vecteur d'expression lui-même dans le vaccin selon l'invention. La présente invention a donc également pour objet tout vecteur d'expression tel que défini ci-dessus. Dans un tel cas de figure, le vecteur d'expression est dépourvu de marqueur et correspond de préférence à un vecteur viral, en particulier un poxvirus tel que ALVAG ou NYVAC.

Toute cellule hôte telle que définie ci-dessus transformée par un tel vecteur d'expression est également incluse dans le cadre de la présente invention.

La présente invention a également pour objet les anticorps dirigés contre les polypeptides et conjugués tels que décrits ci-dessus. La préparation de tels anticorps est mise en oeuvre par les techniques classiques d'obtention d'anticorps polyclonaux et monoclonaux bien connues de l'homme de l'art.

Ces anticorps sont particulièrement appropriés pour être utilisés dans un schéma d'immunisation passive.

La présente invention a également pour objet des compositions pharmaceutiques utiles pour l'induction d'anticorps neutralisants anti-VIH qui sont appropriés pour l'immunisation thérapeutique et prophylactique des infections liées au VIH. Les compositions selon la présente invention comprennent au moins un polypeptide, au moins un conjugué ou au moins un vecteur d'expression tel que défini ci-dessus, un excipient ou diluant pharmaceutiquement acceptable et éventuellement un adjuvant.

Selon un mode de réalisation préféré, la composition selon l'invention comprend en outre un support pharmaceutiquement acceptable. Tout support pharmaceutiquement acceptable susceptible d'adsorber le polypeptide selon l'invention peut être utilisé. Un descriptif de tels supports a été fourni ci-dessus. Dans le cas où le support pharmaceutiquement acceptable est un sel d'aluminium, ce dernier assure à la fois la fonction de support et celle d'adjuvant.

La quantité de polypeptide, de conjugué ou de vecteur dans la composition selon la présente invention dépend de nombreux paramètres comme le comprendra l'homme de l'art, tels que la nature de la protéine porteur, le vecteur utilisé ou, la voie d'administration. Une quantité appropriée est une quantité telle qu'une réponse immunitaire humorale capable de neutraliser des isolats primaires du VIH est induite après administration de cette dernière. La quantité de polypeptide à administrer est de l'ordre de 10µg à 1mg, la quantité sélectionnée variant en fonction de la voie d'administration. La quantité de conjugué à administrer sera déduite des quantités indiquées ci-dessus en tenant compte du PM de la protéine porteur. La quantité de vecteur d'expression à administrer est de l'ordre de 10 à 5000 micro grammes dans le cas d'un vecteur non viral et de l'ordre de 10^{E}4 à 10^{E}8 TCID50 dans le cas d'un vecteur viral.

Les compositions pharmaceutiques selon la présente invention peuvent également contenir un adjuvant. Tout adjuvant ou mélange d'adjuvants pharmaceutiquement acceptable classiquement utilisé dans le domaine des vaccins peut être utilisé à cette fin. On peut citer à titre d'exemple les sels d'aluminium tels que l'hydroxyde d'aluminium ou le phosphate d'aluminium. Des agents auxiliaires classiques tels que agents mouillants, charges, émulsifiants, tampons etc.. peuvent également être additionnés à la composition selon l'invention.

Les compositions selon la présente invention peuvent être préparées par tout procédé classique connu de l'homme de d'art. Classiquement les antigènes selon l'invention sont mélangés avec un excipient ou diluant pharmaceutiquement acceptable, tel que eau ou solution saline tamponnée au phosphate. L'excipient ou diluant va être sélectionné en fonction de la forme galénique choisie, du mode et de la voie d'administration ainsi que de la pratique pharmaceutique. Les excipients ou diluants appropriés ainsi que les exigences en matière de formulation pharmaceutique sont décrits en détails dans Remington's Pharmaceutical Sciences, représentant un ouvrage de référence dans ce domaine.

Les compositions mentionnées ci-dessus peuvent être administrées par toute voie classique, habituellement utilisée dans le domaine des vaccins, telle que la voie parentérale (intraveineuse, intramusculaire, sous-cutanée, etc..). On utilisera de préférence dans le cadre de la présente invention pour les compositions injectables une administration intramusculaire. Une telle administration peut être réalisée avantageusement au niveau des muscles de la cuisse ou du bras. Les compositions selon la présente invention peuvent également avantageusement être administrées par voie orale. Une administration via la muqueuse nasale, vaginale ou rectale peut être également recommandée dans le cadre de la présente invention. L'administration peut être réalisée par l'administration d'une dose unique ou de doses répétées, par exemple à J0, à 1 mois, à 3 mois, à 6 mois et à 12 mois. On utilisera de préférence les injections à J0 , à 1 mois et à 3 mois avec un rappel dont la périodicité pourra être aisément déterminée par le médecin traitant.

La composition pharmaceutique selon la présente invention peut avantageusement être administrée selon un schéma posologique comprenant la co-administration d'un vecteur d'expression selon l'invention et d'un polypeptide selon l'invention ou selon un schéma de « prime-boost » dans lequel le vecteur selon l'invention est administré en premier et le polypeptide en tant qu'injection de rappel. Dans ces deux schémas posologiques, le vecteur d'expression selon l'invention peut être remplacé par tout vecteur d'expression exprimant en outre un ou plusieurs antigènes ou épitopes du VIH différents du polypeptide selon l'invention, et en particulier par un poxvirus de préférence ALVAC ou NYVAC. On peut citer à titre d'exemple de vecteur ALVAC et NYVAC utilisables à cette fin les vecteurs décrits dans les brevets US 5,942,235, US 5,756,103 et US 5,990,091 ; EP 83286, US 5,494,807 et US 5,762,938. On peut également avantageusement utiliser dans le cadre des compositions administrables par voie orale les vecteurs bactériens tels que lactobacillus ou salmonella. L'utilisation de ces vecteurs bactériens à des fins d'immunisation est décrite en détail dans International Journal of Food Microbiology 41 (1998) 155-167 de P.H. Pouwels et al et Cell vol 91, 765-775, Dec, 1997 de A. Darji et al auxquels on pourra se référer pour plus de détails.

La présente invention entend également couvrir un polypeptide, un conjugué ou un vecteur tel que défini ci-dessus et la composition pharmaceutique contenant ces composés pour leur utilisation comme médicament, en particulier pour l'induction d'anticorps neutralisant le VIH à des fins d'immunisation prophylactique et thérapeutique du corps humain contre les infections liées au VIH.

Selon un aspect préféré, la présente invention a pour objet l'utilisation d'un polypeptide selon l'invention pour l'immunisation du corps humain. La présente invention concerne donc de préférence une méthode d'administration dudit polypeptide pour induire une réponse humorale spécifique.

La présente invention concerne donc en particulier une méthode d'induction d'anticorps neutralisant le VIH comprenant l'administration d'une quantité appropriée d'une composition pharmaceutique telle que définie ci-dessus, de préférence d'une composition comprenant un polypeptide de SEQ ID N°28 ou SEQ ID N°31.

On entend par « une réponse humorale spécifique» une réponse comprenant la production d'anticorps dirigés spécifiquement contre le polypeptide selon l'invention. La production d'anticorps spécifiques peut être aisément déterminée par des techniques classiques bien connues de l'homme de l'art telles que ELISA, RIA, western blot.

La demanderesse a mis en évidence de façon surprenante que le polypeptide selon l'invention était capable après administration d'induire des anticorps susceptibles de neutraliser des isolats primaires du VIH. Ces antigènes représentent donc des candidats de valeur pour l'élaboration d'un vaccin utilisable pour la protection et/ou le traitement d'un grand nombre, voire la totalité des sujets à risques ou infectés par le VIH.

Sans vouloir être liée par aucune théorie, la demanderesse pense que la conformation « ouverte » du polypeptide selon l'invention rend accessible des domaines de la gp41 qui ne sont accessibles durant le phénomène de fusion des membranes virale et cellulaire que dans la conformation intermédiaire qui est adoptée de façon transitoire. Ces domaines rendus accessibles constitueraient la cible des anticorps induits par le polypeptide selon l'invention, ces derniers bloquant ainsi le phénomène de fusion membranaire à un stade pré-fusogénique. La conformation ouverte adoptée par le polypeptide selon l'invention mimerait donc la conformation adoptée par l'état intermédiaire.

L'invention a également pour objet une méthode de diagnostic comprenant la mise en contact d'un polypeptide selon l'invention avec un échantillon biologique et la détection des complexes anticorps/polypeptide qui se sont formés. Les sujets VIH+ contiennent en effet des anticorps sériques anti-gp41. Un test immunologique (tel qu'un test ELISA dans lequel le polypeptide selon l'invention est fixé sur la plaque de test puis mis en contact avec le sérum à tester et les complexes anticorps/polypeptide sont ensuite détectés) permettrait donc de diagnostiquer les sujets infectés.

La présente invention va être décrite de façon plus détaillée dans les exemples qui suivent par référence aux figures annexées dans lesquelles :
La figure 1 est une représentation schématique du phénomène de changement de conformation de la gp41 qui précède la fusion des membranes cellulaire et virale.
La figure 2 représente un spectre obtenu par la technique de DSC.
La figure 3 représente un spectre obtenu par la technique de dichroïsme circulaire.
La figure 4 donne la carte plasmidique du pET-Cer (SEQ ID N°35).
Les exemples décrits ci-dessous sont donnés à titre purement illustratif de l'invention et ne peuvent en aucun cas être considérés comme limitant la portée de cette dernière.

### Exemple 1 : Construction des séquences d'ADN codant pour les polypeptides selon l'invention

Les séquences d'ADN codant pour les polypeptides correspondant aux séquences SEQ ID N°28, 29, 30, 31, 32 et 33 ont été obtenues par PCR selon le procédé suivant :

Les séquences N et C sont dans un premier temps amplifiées par PCR à partir d'une matrice correspondant à un plasmide contenant la séquence d'ADN codant pour la région « core »de la gp41 (SEQ ID N° 34) en utilisant les paires d'amorces 5'N/3'N et 5'C/3'C telle que définies ci-dessous dans le tableau 1. Pour chacune des séquences, la réaction de PCR est mise en oeuvre dans les conditions suivantes :
On mélange dans un premier temps les amorces, la matrice, le mélange de nucléotides et la Taq polymérase et on porte le mélange à 94°C pendant 2 minutes. Le mélange résultant est ensuite soumis à 25 cycles de PCR selon le schéma suivant : 94° 30s, 55° 30s, et 68° 30s. Les produits ainsi amplifiés sont ensuite purifiés sur des colonnes QIAQUICK dans les conditions recommandées par le fabricant.

Les deux produits ainsi purifiés qui comportent des séquences chevauchantes sont soumis mélangés et soumis à une réaction d'amplification par PCR utilisant les amorces 5'N et 3'C telles que définies ci-dessous dans le tableau 1. La réaction de PCR est mise en oeuvre selon le schéma suivant : les séquences N et C purifiées, la Taq polymérase et le mélange de nucléotides sont mélangés et soumis à 10 cycles : 94° 30s, 55° 30s, et 68° 30s. On ajoute ensuite les amorces 5'N et 3'C et le mélange résultant est soumis à 20 cycles de PCR selon le schéma suivant : 94° 30s, 55° 30s, et 68° 30s.

Les séquences d'ADN ainsi amplifiées sont ensuite purifiées sur colonnes QIAQUICK dans les conditions recommandées par le fabricant.

**Tableau 1 : Amorces utilisées dans les réaction d'amplification par PCR**

| Construction de SEQ ID N°28 | |
|---|---|
| • 5'N | CAT GCC ATG GCC AGA CAA TTA TTG TCT GG |
| • 3'N | CTC CAT CCA GGT CAT GTT ATT ATC CTT TAG GTA TCT TTC CAC |
| • 5'C | GTG GAA AGA TAC CTA AAG GAT AAT AAC ATG ACC TGG ATG GAG |
| • 3'C | CCG CTC GAG CTA ATG GTG ATG GTG ATG GTG TGA CCC TCC CCC□TCC TTT ATC TAA TTC CAA TAA TTC |

| Construction de SEQ ID N°29 | |
|---|---|
| • 5'N | CAT GCC ATG GCC AGA CAA TTA TTG TCT GG□GTT AAT TTC TCT GTC CCA CTC |
| • 3'N | CAT CCA CTG TTG ATC CTT TAG GTA TC□GAT ACC TAA AGG ATC AAC AGT GGA |
| • 5'C | TGG AGT GGG ACA GAG AAA TTA AC□CCG CTC GAG CTA ATG GTG ATG GTG ATG |
| • 3'C | GTG TGA CCC TCC CCC TCC TTT ATC TAA TTC CAA TAA TTC |

| Construction de SEQ ID N°30 | |
|---|---|
| • 5'N | CAT GCC ATG GCC AGA CAA TTA TTG TCT GG |
| • 3'N | CAT CCA GGT CAT GTT ATT ATC CTT TAG GTA TCT TTC |
| • 5'C | GAA AGA TAC CTA AAG GAT AAT AAC ATG ACC TGG ATG |
| • 3'C | CCG CTC GAG CTA ATG GTG ATG GTG ATG GTG TGA CCC TCC CCC TCC TTT ATC TAA TTC CAA TAA TTC |

| Construction de SEQ ID N°31 | |
|---|---|
| • 5'N | TTA TTG GAA TTA GAT AAA GCC AGA CAA TTA TTG TCT |
| • 3'N | CCG CTC GAG CTA ATG GTG ATG GTG ATG GTG TGA CCC TCC CCC |
| | TCC CTT TAG GTA TCT TTC CAC |
| • 5'C | CAT GCC ATG GGA TGG ATG GAG TGG GAC AGA G |
| • 3'C | AGA CAA TAA TTG TCT GGC TTT ATC TAA TTC CAA TAA |

| Construction de SEQ ID N°32 | |
|---|---|
| • 5'N | GGA ATT AGA TAA ATG GGC AGC CAG ACA ATT ATT GTC TGG |
| • 3'N | CCG CTC GAG CTA ATG GTG ATG GTG ATG GTG TGA CCC TCC |
| | CCC TCC CTT TAG GTA TCT TTC CAC |
| • 5'C | CAT GCC ATG GGA TGGATG GAG TGG GAC AGA G□CCA GAC AAT AAT TGT CTG |
| • 3'C | GCT GCC CAT TTA TCT AAT TCC |

| Construction de SEQ ID N°33 | |
|---|---|
| • 5'N | GGG CAA GTT TGT GGA ATT GGG CCA GAC AAT TAT TGT CTG GCCG CTC GAG |
| • 3'N | CTA ATG GTG ATG GTG ATG GTG TGA CCC TCC CCCTCC CTT TAG GTA TCT TTC |
| | CAC |
| • 5'C | CAT GCC ATG GGA TGG ATG GAG TGG GAC AGA GCCA GAC AAT AAT TGT CTG |
| • 3'C | GCC CAA TTC CAC AAA CTT GCC C |

### Exemple 2 : Clonage des séquences d'ADN de l'exemple 1 dans un vecteur d'expression.

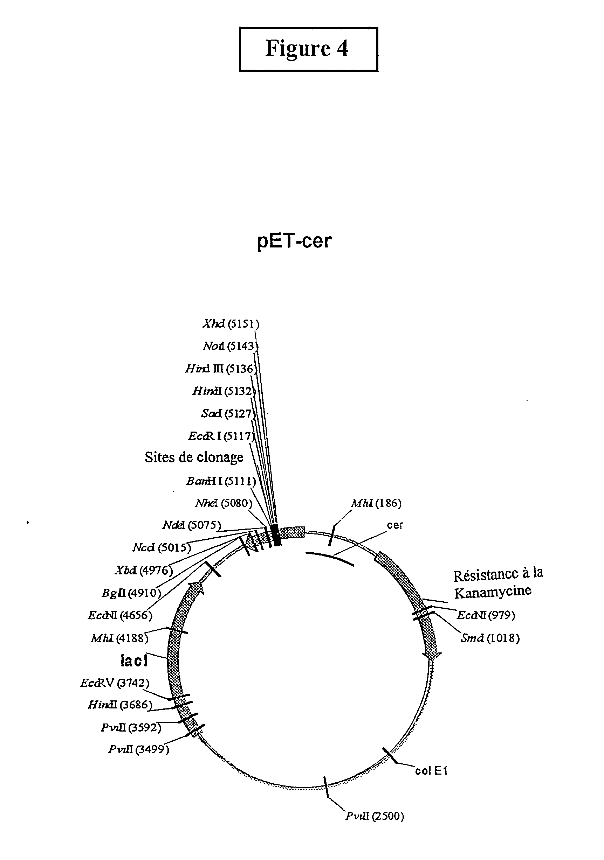

Les séquences d'ADN produites dans l'exemple 1 sont digérées par NcoI et XhoI dans des conditions standards en utilisant 10 unités de chacune des enzymes pour 1µg de fragment PCR, puis clonées dans un vecteur pET-cer selon le mode opératoire suivant : le fragment PCR et le vecteur digéré par les enzymes NcoI et XhoI sont ligués entre eux puis utilisés pour transformer une souche bactérienne XL1 par électroporation. L'obtention de la construction est vérifiée par minipréparation du plasmide et séquençage.
Le vecteur pET-cer utilisé est construit à partir du vecteur pET28 de Novagen. Le vecteur commercial pET28c a été amplifié par PCR à l'aide de 2 amorces situées de part et d'autre de la région correspondant à l'origine f1, de sorte que le produit amplifié corresponde à la quasi totalité du vecteur d'origine moins la région comprenant l'origine f1. Les sites de restriction uniques AscI et PacI sont apportés respectivement par les 2 amorces ayant servi à l'amplification. Parallèlement le fragment cer est amplifié à l'aide de 2 amorces qui permettent d'obtenir ce fragment encadré par les sites AscI et PacI.
Vecteur et fragment cer sont digérés par les enzymes AscI et PacI puis ligués entre eux Ce vecteur comprend en particulier une cassette d'expression sous le contrôle du promoteur T7, un polylinker en aval du promoteur T7 pour le clonage du gène d'intérêt, le fragment cer situé en aval du polylinker , permettant de diminuer la multimérisation des plasmides, un terminateur de transcription T7 term et le gène de résistance à la kanamycine.
La régulation positive du promoteur est obtenue en présence de la T7 RNA polymérase. La carte plasmidique du plasmide pET-cer est donnée à la figure 4 (SEQ ID N° 35).

### Exemple 3 : Préparation du polypeptide SEQ ID N°31 selon l'invention

### 1- Expression

L'expression du plasmide issu de l'exemple 2 renfermant la séquence codant pour le polypeptide SEQ ID N°31 est réalisée chez une souche modifiée d' E.coli i.e. :BL21 RILλDE3.
Cette souche est enrichie en tRNA rares (ARG, ILE, LEU), elle contient le gène codant pour la T7 RNA polymérase lequel est sous le contrôle du promoteur lac UV5 inductible par addition d'IPTG à une concentration de 1mM.
Dans un premier temps la souche est transformée par le plasmide selon le protocole comprenant les étapes suivantes : repiquage de 3 colonies dans 10 ml de LB additionné de kanamycine à une concentration de 25µg/µl; Incubation une nuit à 37°C ; réensemencer la préculture au 1:100 dans 15ml de LB additionné de kanamycine à une concentration de 25µg/µl; laisser pousser jusqu'à DO600 de 0,5 ; Prélever 1 ml pour vérifier la DO600 ; prélever 7 ml pour l'échantillon non induit ; induire les 7 autres ml avec 1mM d'IPTG et induction 3H à 37°C.
Le même protocole a été mis en oeuvre sur plusieurs litres de culture pour produire une grande quantité de bactéries pour purifier le polypeptide selon l'invention.

### 2- Purification

le culot cellulaire, formé des bactéries récoltées dans 500ml de milieu de culture, est décongelé et repris dans 100ml de tampon Tris-HCl 50mM à pH8,0 en présence d'un inhibiteur de protéase (Péfabloc, Interchim) à la concentration de 100µM. Du lysozyme est ajouté à la concentration de 100µg/ml et le mélange est incubé 30 minutes à température ambiante avec agitation. Les cellules sont ensuite cassées par sonication (4 cycles de deux minutes) avec une puissance approximative de 150 Watts. On ajoute ensuite de la benzonase (DNase, Merck) avec du MgCl2 1mM et on incube le mélange 20 min à température ambiante avec agitation. Après centrifugation (20 min à 10000g), le polypeptide selon l'invention se trouve dans la fraction insoluble sous forme de corps d'inclusion. Ceux-ci sont lavés avec du tampon Tris-HCl 50mM à pH8,0 et centrifugés pendant 15min à 10000g.
Le polypeptide selon l'invention peut ensuite être purifié par l'un des deux procédés suivant :

Procédé 1 : Après élimination du surnageant, la solubilisation du culot de centrifugation composé essentiellement des corps d'inclusion est effectuée en une heure à température ambiante par agitation douce en présence de 50ml de tampon CAPS à pH 11,0 contenant 1% de N-lauryl sarcosine.
La fraction solubilisée est ensuite dialysée à 4°C contre du tampon Tris-HCl 50mM à pH 8,0 contenant des concentrations décroissantes de détergent (0.2% final) et filtrée sur un filtre de porosité 0,45µm puis chargée sur une colonne Hi-Trap 1ml (Pharmacia). Ces supports de chromatographie d'affinité chélatent des atomes de Nickel sur lesquels se fixent les résidus histidine de l'extrémité C-terminal du polypeptide. Après lavage, l'élution du polypeptide est obtenue avec du tampon acide formique 50mM à pH 2,5.

Procédé 2 : Après élimination du surnageant, la solubilisation du culot de centrifugation composé essentiellement des corps d'inclusion est effectuée en une heure à température ambiante par agitation douce en présence de 30ml de tampon. Tris-HCl 50mM pH8,0, urée 8M. Après filtration sur un filtre de porosité 0,45µm, la fraction est chargée sur une colonne Hi-Trap 1ml (Pharmacia). Après lavage, l'élution est réalisée avec du tampon Tris-HCl 50mM pH8,0 + Urée 8M + imidazole 500mM. La fraction éluée est ensuite dialysée contre du tampon acide formique 50mM à pH 2,5.

### Exemple 4 : Préparation du polypeptide SEQ ID N°30 selon l'invention:

Le polypeptide est exprimé en utilisant le protocole décrit dans l'exemple 3 puis purifié selon le mode opératoire décrit ci-dessous.
Le culot cellulaire, formé des bactéries récoltées dans 500ml de milieu de culture, est décongelé et repris dans 100ml de tampon Tris-HCl 50mM à pH8,0 en présence d'un inhibiteur de protéase (Péfabloc, Interchim) à la concentration de 100µM. Du lysozyme est ajouté à la concentration de 100µg/ml et le mélange est incubé 30 minutes à température ambiante avec agitation. Les cellules sont ensuite cassées par sonication (4 cycles de deux minutes) avec une puissance approximative de 150 Watts. On ajoute ensuite de la benzonase (DNase, Merck) avec du MgCl2 1 mM et on incube le mélange 20 min à température ambiante avec agitation. Après centrifugation (20 min à 10000g), le polypeptide selon l'invention se trouve majoritairement dans la fraction soluble. Après filtration sur un filtre de porosité 0,45µm, la fraction est chargée sur une colonne Hi-Trap 1ml (Pharmacia). Après lavage, l'élution est réalisée avec du tampon Tris-HCl 50mM pH8,0 + imidazole 500mM. La fraction éluée est ensuite dialysée contre du tampon tampon Tris-HCl 50mM pH 8,0.
Le polypeptide à une concentration de 0,4mg/ml est ensuite placé dans un tampon formate de sodium 50mM à pH 2,5. Un mélange TFE/polypeptide est préparé par addition de 1 vol. de TFE pour 1 vol. de solution de polypeptide. Le mélange ainsi obtenu est incubé à température ambiante pendant 15 min. 6mg de phosphate d'A1 sont ajoutés ainsi que du tampon formate de sodium 50mM pour obtenir un volume final de 0,5 ml.
Le mélange est ensuite incubé à 4°C sous agitation pendant un temps qui peut varier entre 15 min. et 3 heures de façon à permettre l'adsorption totale de la protéine sur le phosphate d'aluminium. Après l'étape d'adsorption, le mélange est centrifugé pendant 5 minutes à environ 10000g. Le surnageant est éliminé et le culot est resuspendu dans le volume initial en ajoutant la quantité nécessaire de tampon PBS. Une deuxième centrifugation et une reprise dans le même tampon permet d'éliminer les dernières traces d'acide formique et de TFE.
La préparation ainsi obtenue forme la composition pharmaceutique administrable.

### Exemple 5 : Analyse par DSC des polypeptides selon l'invention

Les analyses ont été réalisées sur un microcalorimètre VP (Microcal, Northampton, Massachusetts, USA) de la façon suivante :
Préparation des échantillons :
Concentration en polypeptide : 0,4-0,7mg/ml, déterminée par la méthode MicroBCA (Pierce, Rockford, Illinois, USA). Le polypeptide est filtré avant analyse (seuil de coupure de 0,22µm), dégazé pendant 8 min. dans le Thermovac (Microcal, Northampton, Massachusetts, USA) puis thermostaté à 24°C. Les échantillons sont en tampon formate de Na 50mM pH2,5.
Enregistrement :
Après l'échauffement de l'appareil qui consiste en 6-10 thermocycles du tampon contre tampon, le polypeptide est placé dans la cellule de mesure quand la température de la cellule est de 25°C.
Les paramètres des thermocycles sont les suivants : (1) 15 min. d'équilibrage à 5°C; (2) augmentation de la température de 5°C-130°C avec une vitesse de 85°C/H ; (3) 3 min d'équilibrage à 130°C et (4) refroidissement de 130°C à 5°C (vitesse maximale) comprenant l'étape de chargement de l'échantillon suivant à la température de 25°C pour la mesure suivante.

L'analyse a été réalisée sur l'ectodomaine de la gp41 LAI constitué des acides aminés AA25-AA157 et sur les polypeptides SEQ ID N°31 et SEQ ID N°30 selon l'invention.
La concentration protéique était de 0,51 mg/ml pour l'ectodomaine entier de gp41 et de 0,63 mg/ml pour les polypeptides selon l'invention.

Les résultats représentés à la figure 2 sont donnés dans le tableau 2 ci-dessous :

**Tableau 2 :**

| Echantillon | Tm (°C) | ΔH | ΔH_{VH} |
|---|---|---|---|
| | [écart type 0,2°C] | (Kcal/mol) | (Kcal/mol) |
| Ectodomaine entier de gp41 | 110, 4 | 61 | 228 |
| (-) | | | |
| Polypeptide SEQ ID N°3 | 101,9 | 58 | 140 |
| (-..-..-..-) | | | |
| Polypeptide SEQ ID N°30 | 90,3 | 59 | 152 |
| (..........) | | | |

### Exemple 6 : Analyse par dichroïsme circulaire des polypeptides selon l'invention

Les analyses ont été réalisées sur un spectropolarimètre (Jasco, Tokyo, Japan) dans les conditions suivantes : temp : 25°C ; Cuve : trajet optique :0,1mm ; Concentration en polypeptide : 1-2 mg/ml. Le polypeptide est placé dans la cuve et le spectre est enregistré à une vitesse de balayage de 10nm/min. Les spectres obtenus sont analysés par le logiciel Spectra Analysis de Jasco. Le spectre du tampon est utilisé pour corriger le bruit de fond.

L'analyse a été réalisé sur l'ectodomaine entier de la gp41 LAI (i.e. AA25-157) et sur le polypeptide SEQ ID N°31 selon l'invention.

Les résultats représentés à la figure 3 montrent que l'ectodomaine entier présente un taux d'hélicité de 69%, le polypeptide selon l'invention présente un taux d'hélicité de 70% et le polypeptide mélangé au TFE présente un taux d'hélicité de 100%.

### Exemple 7 : Détermination de l'immunité humorale induite par les polypeptides selon l'invention

Les polypeptides SEQ N° 28, N° 29, N° 31 et N°32 ont été testés chez le cobaye, chez le lapin et chez le macaque Cynomolgus selon les protocoles décrits ci-dessous.
✔ Cobayes : des groupes de 5 cobayes ont été injectés 3 fois, à 3 semaines d'intervalle, dans les cuisses (muscle *biceps femoris)* avec 20 µg par dose d'antigène. A chaque injection, les animaux ont reçu 0,5 ml de la formulation (0,25 ml dans chaque cuisse).

Les sérums des animaux ont été prélevés pour l'analyse des anticorps avant immunisation, puis 3 et 2 semaines après les 2^{nde} et 3^{ème} immunisations, respectivement.

Les deux compositions testées : antigène + alum (phosphate d'aluminium, 6 mg par dose) et antigène + alum (phosphate d'aluminium, 6 mg par dose) + TFE ont été préparées de la façon suivante :
Pour les formulations adjuvées avec le phosphate d'aluminium : l'antigène se trouve en milieu formate 50mM, pH 2.5. Les formulations sont obtenues par ajout de l'alum à la composition d'antigène et incubation sous agitation douce pendant 30 minutes. le mélange est ensuite centrifugé (5 minutes à 3000 rpm), le surnageant étant prélevé et remplacé par du tampon PBS de sorte à obtenir 500µl/dose en final. La remise en suspension est réalisée à l'aide d'un bain à ultrasons.
Pour la formulation avec phosphate d'aluminium et TFE : on ajoute à l'antigène qui se trouve en milieu formate 50mM, pH 2.5 un volume de TFE (50% en vol. au final). Le mélange est incubé environ 15 minutes à température ambiante et 6 mg de phosphate d'aluminium ainsi que du tampon formate sont rajoutées pour ajuster le volume à 0,5 ml correspondant au volume injecté. Le mélange ainsi obtenu est ensuite incubé à 4°C sous agitation pendant une période pouvant varier entre 15 minutes et 3 heures pour permettre l'adsorption de l'antigène sur le phosphate d'aluminium. Après incubation, le mélange est centrifugé pendant 5 minutes à 10000g environ et le culot est remis en suspension dans du tampon PBS. Cette étape est réalisée deux fois pour éliminer toute trace de TFE. Le culot est remis en suspension à l'aide d'un bain à ultrasons dans le volume injectable.

✔ Lapins : des groupes de 2 lapins ont été injectés 3 fois, à 3 semaines d'intervalle, dans les cuisses avec 40 µg par dose d'antigène. A chaque injection, les animaux ont reçu 1 ml de la formulation.

Les sérums des animaux ont été prélevés pour analyses anticorps avant immunisation, puis 3 et 2 semaines après les 2^{nde} et 3^{ème} immunisations, respectivement.

La composition testée ici : antigène + alum (phosphate d'aluminium, 6 mg par dose); a été préparée de la façon suivante : à l'antigène, en milieu formate 50mM, pH 2.5, est ajouté du phosphate d'aluminium, l'ensemble étant incubé 30 minutes à +4°C sous faible agitation (roue mobile). Les tubes contenant ces préparations sont ensuite centrifugés (5 minutes à 3000 rpm), le surnageant étant prélevé et remplacé par du tampon PBS de sorte à obtenir 1 ml/dose en final. La remise en suspension est réalisée à l'aide d'un bain à ultrasons.
✔ Macaques Rhesus (*macaca fascicularis*) : des groupes de 2 macaques ont été injectés 3 fois à 1 mois d'intervalle dans les cuisses (muscle *rectus femoris*) avec 100 µg par dose d'antigène adsorbés sur 6 mg d'alum (phosphate d'aluminium). A chaque injection, les animaux ont reçu 1 ml de la formulation.

Les sérums des animaux ont été prélevés pour analyses anticorps avant immunisation, puis 4 et 2 semaines après les 2^{nde} and 3^{ème} immunisations, respectivement.

La composition testée ici : antigène + alum a été préparée selon le protocole utilisé pour les expériences chez le lapin.

Les résultats sont donnés dans les tableaux ci-dessous :
Comme le montre le tableau 1, les polypeptides selon l'invention induisent des taux d'anticorps ELISA significatifs, homogènes et spécifiques contre l'ectodomaine de la protéine gp41 LAI ( exprimé chez E. coli sous forme d'un trimère adoptant une conformation fusogène) et contre la gp160 MN/LAI-2 (glycoprotéine hybride dans laquelle la sous-unité gp120 dérive de l'isolat VIH-1 MN et la sous-unité gp41 dérive de l'isolat VIH-1 LAI). Ces réponses IgG atteignent quasiment un plateau dès la 2^{ème} injection (tableau 1).

**Tableau 1- Test cobaye - Réponses anticorps en ELISA**

| **Immunogène** | **IgG anti-ectodomaine gp41** | | **IgG anti-gp160 MN/LAI-2** | |
|---|---|---|---|---|
| | **Titres IgG (log₁₀) post-2*** | **Titres IgG (log₁₀) post-3*** | **Titres IgG (log₁₀) post-2*** | **Titres IgG (log₁₀) post-3*** |
| | *(nombre de positifs)* | *(nombre de positifs)* | *(nombre de positifs)* | *(nombre de positifs)* |
| **SEQ ID N°28** | **5.1** ± *0.2* | **5.1** ± *0.2* | **4.7** ± *0.4* | **5.0** ± *0.4* |
| *(alum)* | *(5+*/*5)* | *(5+*/*5)* | *(5+*/*5)* | *(5+*/*5)* |
| **SEQ ID N°28** | **5.4** *± 0.1* | **5.3** ± *0.2* | **4.7** ± *0.2* | **5.0** ± *0.2* |
| *(alum) +* **TFE** | (*5+*/*5*) | (*5+*/*5*) | (*5+*/*5*) | (*5+*/*5*) |
| **SEQ ID N°31** | **5.1** ± *0.2* | **5.2** ± *0.2* | **5.0** ± *0.1* | **5.3** ± *0.2* |
| *(alum)* | (5+/5) | (5+/5) | (5+/5) | (5+/5) |
| **SEQ ID N°31** | **5.2** ±*0.1* | **5.1** ±*0.1* | **4.7** ± *0.3* | **4.9** ± *0.1* |
| *(alum) +* **TFE** | *(4+*/*4)* | *(4+*/*4)* | *(4+*/*4)* | *(4+*/*4)* |
| **SEQ ID N°32** | **5.0** ± *0.1* | **NT** | **4.5** ± *0.3* | **NT** |
| *(alum)* | *(5+*/*5)* | | *(5+*/*5)* | |

| | | | | |
|---|---|---|---|---|
| * **Moyenne géométrique** ± *déviation standard* (log₁₀) NB : tous les sérums préimmuns testés sont inférieurs au seuil de détection (soit 1.9 log₁₀ pour l'ELISA anti-gp160 et 1.0 log₁₀ pour l'ELISA anti-ectodomaine). | | | | |

L'activité neutralisante des sérums post-3° immunisation a ensuite été évaluée vis à vis de la souche de laboratoire VIH-1 MN sur des sérums individuels (chez DC Montefiori). Comme le montrent les résultats obtenus, aucune neutralisation de la souche MN n'a été observée

L'activité neutralisante des sérums post-3 a également été évaluée vis à vis de souches de VIH-1 primaires (laboratoires de C. Moog et de D. Montefiori). L'analyse a été réalisée sur des sérums individuels. De manière intéressante, contrairement à ce qui a été observé pour la souche MN, les cobayes ont montré des activités neutralisantes significatives contre plusieurs des souches virales testées. Les résultats obtenus sont consignés dans le tableau 2.

**Tableau 2 - Test cobaye - Réponses anticorps neutralisantes anti-VIH-1**

| **Immunogène** | **Souche de laboratoire** | **Isolats primaires** | | | | | |
|---|---|---|---|---|---|---|---|
| | **MN§** | **Bal§** | **SF162§** | **5768§** | **Pavo§** | **Bx08¤** | **Bx17¤** |
| **SEQ ID N°31** *(alum)* | ≤ 0.55 | **97%** | **88%** | **97%** | **95%** | **4** | NT |
| **SEQ ID N°31** *(alum)* **+ TFE** | ≤ 0.55 | **93%** | NT | **88%** | **86%** | **4** | **4** |
| **SEQ ID N°28** *(alum)* | ≤ 0.55 | **93%** | NT | **92%** | NT | **10** | **10** |

| | | | | | | | |
|---|---|---|---|---|---|---|---|
| § Lab. D. Montefiori : résultats donnés pour les sérums post-3 (valeur arithmétique ou %) ¤ Lab. C. Moog : résultats donnés pour les sérums post-3 (valeur arithmétique) NT : non testé NB : tous les sérums préimmuns testés sont inférieurs au seuil de positivité (soit, suivant les méthodes : < 20 pour la souche VIH-1 MN et < 80% ou < 4 pour les isolats primaires). | | | | | | | |

**Tableau 3 - Test lapin - Réponses anticorps ELISA**

| **Immunogène** | **IgG anti-ectodomaine gp41** | |
|---|---|---|
| | **Titres IgG (log₁₀) post-2*** | **Titres IgG (log₁₀) post-3*** |
| | *(nombre de positifs)* | *(nombre de positifs)* |
| **SEQ ID N°28** | **4.6** ± 0.2 | **4.8** ± 0.2 |
| *(alum)* | *(2*+/*2)* | *(*2+/2*)* |
| **SEQ ID N°31** | **4*.*5** ± *0.2* | **4.8** ± *0.2* |
| *(alum)* | *(*2+/2*)* | *(*2+/2*)* |

**Tableau 4 - Test macaque - Réponses anticorps ELISA**

| **Immunogène** | **IgG anti-ectodomaine gp41** | |
|---|---|---|
| | **Titres IgG (log₁₀) post-2*** | **Titres IgG (log₁₀) post-3*** |
| | *(nombre de positifs)* | *(nombre de positifs)* |
| **SEQ ID N°29** | **5,1** ± *0*.*0* | **5.4** ± *0*.*0* |
| *(alum)* | *(2+*/*2)* | *(2+*/*2)* |
| **SEQ ID N°28** | **4.7** ± *0*.*2* | **5.2** ± *0*.*2* |
| *(alum)* | *(2+*/*2)* | *(2+*/*2)* |
| **SEQ ID N°31** | **4.9** ± *0*.*7* | **5.4** ± *0*.*3* |
| *(alum)* | *(2+*/*2)* | *(2+*/*2)* |
| **SEQ ID N°32** | **5.0** ± *0*.*1* | **5.2** ± *0*.*2* |
| *(alum)* | *(2+*/*2)* | *(2*+/*2)* |

| | | |
|---|---|---|
| NB : les sérums préimmuns testés s'avèrent sont inférieurs au seuil de positivité (1.0 log₁₀). | | |

Les résultats donnés ci-dessus montrent clairement que le polypeptide selon l'invention est capable d'induire des taux d'anticorps ELISA spécifiques significatifs chez toutes les espèces animales testées. Ces réponses IgG ont augmenté faiblement entre la 2^{ème} et la 3^{ème} injection.

Ces anticorps qui ont la propriété de neutraliser les isolats primaires font du polypeptide selon l'invention un candidat de valeur pour une immunisation chez l'homme.

## Revendications

1. Polypeptide comprenant une séquence de formule I :
[N-S1]n-C-[S2-N]m
dans laquelle :
N représente la séquence des acides aminés 30 à 77 de la protéine gp41, du virus VIH-I
C représente la séquence des acides aminés 117 à 154 de la protéine gp41, du virus VIH-I
S1 est absent ou représente la séquence d'acides aminés D, DQ, DQQ, DQQL ou DNNMT,
S2 est absent ou représente la séquence d'acides aminés W, WA, WAS, WASL ou WASLW, et
n = 0 ou 1 ; m = 0 ou 1 et m + n = 1 ou 2.

2. Polypeptide selon la revendication 1 dans lequel m+n=1.

3. Polypeptide selon la revendication 1 ou 2 dans lequel N représente SEQ ID N°26 et C représente SEQ ID N°27.

4. Polypeptide selon l'une quelconque des revendications 1 à 3 sélectionné dans le groupe consistant en SEQ ID N°28 et SEQ ID N°31.

5. Polypeptide selon l'une quelconque des revendications 1 à 4 comprenant une séquence supplémentaire de formule (G)a-S-(H)b dans laquelle G représente un résidu glycine, H représente un résidu histidine, a est supérieur ou égal à 4 et b est supérieur ou égal à 6, ladite séquence étant liée par liaison amide à l'extrémité NH₂- ou COOH-terminale du polypeptide.

6. Conjugué comprenant un polypeptide selon l'une quelconque des revendications 1 à 5, conjugué à une protéine ou un peptide porteur.

7. Séquence d'ADN codant pour un polypeptide selon l'une quelconque des revendications 1 à 5 ou pour un conjugué selon la revendication 6.

8. Vecteur d'expression comprenant la séquence d'ADN selon la revendication 7.

9. Cellule hôte contenant le vecteur selon la revendication 8.

10. Procédé de préparation d'un polypeptide selon l'une quelconque des revendications 1 à 5 comprenant l'expression dudit polypeptide à partir d'une cellule hôte telle que définie dans la revendication 9.

11. Composition pharmaceutique comprenant au moins un polypeptide selon l'une des revendications 1 à 5, ou au moins un conjugué selon la revendication 6 ou au moins un vecteur d'expression selon la revendication 8, un excipient pharmaceutiquement acceptable et éventuellement un adjuvant.

12. Composition pharmaceutique selon la revendication 11 administrable par voie orale.

13. Composition pharmaceutique selon la revendication 11 ou 12 comprenant en outre un support pharmaceutiquement acceptable.

14. Polypeptide selon l'une quelconque des revendications 1 à 5 pour son utilisation à titre de médicament.

15. Polypeptide selon la revendication 14 pour l'immunisation du corps humain contre les infections liées au VIH.

## Claims

1. - Polypeptide comprising a sequence of formula I:
[N-S1]n-C-[S2-N]m
in which:
N represents the sequence of amino acids 30 to 77 of the gp41 protein of the HIV-1 virus,
C represents the sequence of amino acids 117 to 154 of the gp41 protein of the HIV-1 virus,
S1 is absent or represents the amino acid sequence D, DQ, DQQ, DQQL or DNNMT,
S2 is absent or represents the amino acid sequence W, WA, WAS, WASL or WASLW, and
n = 0 or 1; m = 0 or 1 and m + n = 1 or 2.

2. - Polypeptide according to Claim 1, in which m + n=1.

3. - Polypeptide according to either one of Claims 1 and 2, in which N represents SEQ ID No. 26 and C represents SEQ ID No. 27.

4. - Polypeptide according to any one of Claims 1 to 3, selected from the group consisting of SEQ ID No. 28 and SEQ ID No. 31.

5. - Polypeptide according to any one of Claims 1 to 4, comprising an additional sequence of formula (G)a-S-(H)b in which G represents a glycine residue, H represents a histidine residue, a is greater than or equal to 4 and b is greater than or equal to 6, said sequence being linked via an amide bond to the NH₂- or COOH-terminal end of the polypeptide.

6. - Conjugate comprising a polypeptide according to any one of Claims 1 to 5, conjugated to a carrier protein or peptide.

7. - DNA sequence encoding a polypeptide according to any one of Claims 1 to 5 or a conjugate according to Claim 6.

8. - Expression vector comprising the DNA sequence according to Claim 7.

9. - Host cell containing the vector according to Claim 8.

10. - Method for preparing a polypeptide according to any one of Claims 1 to 5, comprising the expression of said polypeptide using a host cell as defined in Claim 9.

11. - Pharmaceutical composition comprising at least one polypeptide according to any one of Claims 1 to 5, at least one conjugate according to Claim 6 or at least one expression vector according to Claim 8, a pharmaceutically acceptable excipient and, optionally, an adjuvant.

12. - Pharmaceutical composition according to Claim 11, which can be administered orally.

13. - Pharmaceutical composition according to Claim 11 or 12, also comprising a pharmaceutically acceptable support.

14. - Polypeptide according to any one of Claims 1 to 5, for its use as a medicinal product.

15. - Polypeptide according to Claim 14, for immunization of the human body against HIV-related infections.

## Patentansprüche

1. Polypeptid, umfassend eine Sequenz der Formel I
[N-S1]n-C-[S2-N]m
wobei N die Sequenz der Aminosäuren 30-77 des gp41-Proteins des HIV-1-Virus darstellt,
C die Sequenz der Aminosäuren 117-154 des gp41-Proteins des HIV-1-Virus darstellt,
S1 fehlt oder die Sequenz der Aminosäuren D, DQ, DQQ, DQQL oder DNNMT darstellt,
S2 fehlt oder die Sequenz der Aminosäuren W, WA, WAS, WASL oder WASLW darstellt und
n = 0 oder 1 bedeutet; m = 0 oder 1 bedeutet und m + n = 1 oder 2 bedeutet.

2. Polypeptid nach Anspruch 1, wobei m + n = 1 bedeutet.

3. Polypeptid nach Anspruch 1 oder 2, wobei N SEQ ID NO: 26 darstellt und C SEQ ID NO: 27 darstellt.

4. Polypeptid nach einem der Ansprüche 1 bis 3, ausgewählt aus der Gruppe, bestehend aus SEQ ID NO: 28 und SEQ ID NO: 31.

5. Polypeptid nach einem der Ansprüche 1 bis 4, umfassend eine zusätzliche Sequenz der Formel (G)a-S-(H)b, wobei G einen Glycinrest darstellt, H einen Histidinrest darstellt, a größer oder gleich 4 ist und b größer oder gleich 6 ist, wobei die Sequenz über eine Amidbindung mit dem NH₂- oder COOH-Terminus des Polypeptids verknüpft ist.

6. Konjugat, umfassend ein Polypeptid nach einem der Ansprüche 1 bis 5, das mit einem Trägerprotein oder einem Trägerpeptid konjugiert ist.

7. DNA-Sequenz, die ein Polypeptid nach einem der Ansprüche 1 bis 5 oder ein Konjugat nach Anspruch 6 codiert.

8. Expressionsvektor der die DNA-Sequenz nach Anspruch 7 umfasst.

9. Wirtszelle, die den Vektor nach Anspruch 8 enthält.

10. Verfahren zur Herstellung eines Polypeptids nach einem der Ansprüche 1 bis 5, umfassend die Expression des Polypeptids ausgehend von einer Wirtszelle, wie in Anspruch 9 definiert.

11. Pharmazeutische Zubereitung, umfassend mindestens ein Polypeptid nach einem der Ansprüche 1 bis 5 oder mindestens ein Konjugat nach Anspruch 6 oder mindestens einen Expressionsvektor nach Anspruch 8, ein pharmazeutisch verträgliches Exzipiens und gegebenenfalls ein Adjuvans.

12. Pharmazeutische Zubereitung nach Anspruch 11, die oral verabreichbar ist.

13. Pharmazeutische Zubereitung nach Anspruch 11 oder 12, die weiterhin einen pharmazeutisch verträglichen Träger umfasst.

14. Polypeptid nach einem der Ansprüche 1 bis 5 für seine Verwendung als Medikament.

15. Polypeptid nach Anspruch 14 zur Immunisierung des menschlichen Körpers gegen die mit HIV verbundenen Infektionen.
